# EUROPEAN PATENT APPLICATION

(11) **EP 4 793 272 A1**
(43) Date of publication of application: **19.08.2026**
(21) Application number: 24877620.5
(22) Date of filing: 10.10.2024
(51) Int. Cl.: C07D 251/24, C07D 405/04, C07D 239/26, C07D 409/04, H10K 85/60

(54) **ORGANIC COMPOUND AND ORGANIC ELECTROLUMINESCENT DEVICE USING SAME**

(30) Priority: 11.10.2023 KR 20230135444
(71) Applicant: Solus Advanced Materials Co., Ltd., Iksan-si, Jeollabuk-do 54584 (KR)
(72) Inventor: CHO, Sukwon, Seongnam-si, Gyeonggi-do 13636 (KR); SIM, Jaeyi, Seongnam-si, Gyeonggi-do 13636 (KR); PARK, Woojae, Seongnam-si, Gyeonggi-do 13636 (KR); SHIN, Seongmin, Seongnam-si, Gyeonggi-do 13636 (KR)
(74) Representative: Office Freylinger
(86) International application number: PCT/KR2024/096282
(87) International publication number: WO 2025/080085

(57) **Abstract**

The present invention relates to a novel compound with excellent carrier transport ability, light-emitting ability, and thermal stability and an organic electroluminescent device comprising same in one or more organic layers thus having improved properties of luminous efficiency, driving voltage, lifespan, etc.

## Description

### Technical Field

The present invention relates to a novel organic compound and an organic electroluminescent device using the same and, more specifically, to a novel compound with excellent electron transporting ability, and an organic electroluminescent device exhibiting improved characteristics, such as luminous efficiency, driving voltages, and lifespan, by containing the novel compound in one or more organic layers.

### Background Art

In an organic electroluminescent device, upon the application of voltage between two electrodes, holes from an anode and electrons from a cathode are injected into organic layers. The injected holes and electrons combine with each other to form excitons, and the excitons fall down to the ground state to emit light. Particularly, materials used for the organic layers may be classified into light emission materials, hole injection materials, hole transport materials, electron transport materials, electron injection materials, and the like according to the function thereof.

Materials for forming an emissive layer of the organic electroluminescent device may be classified into blue, green and red light emission materials according to the color of light emission. Additionally, yellow and orange light emission materials may be used as light emission materials for displaying better natural colors. Additionally, host/dopant-based light emission materials may be used as light emission materials to increase color purity and improve luminous efficiency through energy transfer.

Dopant materials may be classified into fluorescent dopants using organic materials and phosphorescent dopants using metal complex compounds containing heavy atoms, such as Ir and Pt. These phosphorescent materials can theoretically improve the luminous efficiency up to four times compared to fluorescent materials, so research has been conducted on phosphorescent host materials as well as phosphorescent dopants.

Until today, NPB, BCP, Alq3, and the like have been widely known as materials for use in a hole injection layer, a hole transport layer, and an electron transport layer, and anthracene derivatives have been reported as the light emitting layer materials. In particular, among the light emitting layer materials, metal complex compounds containing Ir, such as Firpic, Ir(ppy)₃, (acac)Ir(btp)₂, and the like, which have advantages in terms of improving efficiency, are used as blue, green, and red phosphorescent dopant material, and 4,4-dicarbazollybiphenyl (CBP) is used as the phosphorescent host material.

However, conventional light emission materials have advantages in terms of light emission characteristics, but are not satisfactory in terms of lifespan of organic electroluminescent devices due to low glass transition temperatures and very poor thermal stability. Accordingly, there is a need to develop light emission materials with excellent performance.

### Disclosure of Invention

### Technical Problem

An aspect of the present invention is to provide a novel compound that can be used as a material for an organic layer of an organic electroluminescent device and has excellent hole injection and transport properties, electron injection and transport properties, and light-emitting properties.

Another aspect of the present invention is to provide an organic electroluminescent device exhibiting a low driving voltage, high luminous efficiency, and improved lifespan by containing the above-described novel compound.

Other purposes and advantages of the present invention will be clarified by following detailed description and claims.

### Solution to Problem

To achieve the above objectives, the present invention provides a compound represented by the following Chemical Formula 1: 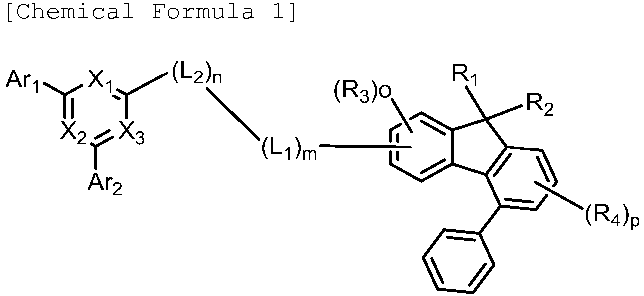 wherein in Chemical Formula 1,
X₁ to X₃ are the same as or different from each other and are each independently N or C(R₅), provided that at least two of X₁ to X₃ are N;
Ar₁ to Ar₂ are the same as or different from each other and are each independently selected from the group consisting of a hydrogen, a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group,
R₁ and R₂ are the same as or different from each other and are each independently selected from the group consisting of a hydrogen, a deuterium (D), a C₁-C₄₀ alkyl group, or a C₆-C₆₀ aryl group, or are bonded to each other to form a fused ring,
R₃ to R₅ are the same as or different from each other and are each independently selected from the group consisting of a hydrogen, a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group, or may be bonded to any adjacent group to form a fused ring,
L₁ and L₂ are the same as or different from one another and are each independently a single bond, or selected from the group consisting of a C₆ to C₁₈ arylene group and a heteroarylene group having 5 to 18 nuclear atoms,
n and m are each independently an integer of 0 to 2, provided that n + m ≥ 1, and
o and p are each independently an integer of 0 to 3, and
the arylene group, the heteroarylene group of L₁ and L₂; and the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, and arylamine group of Ar₁ to Ar₂, and R₁ to R₅ may be each independently substituted with at least one substituent selected from the group consisting of a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and wherein when the substituent is present in a plural number, they may be the same or different from each other.

In addition, the present invention also provides an organic electroluminescent device including: an anode, a cathode, and one or more organic layers disposed between the anode and the cathode, wherein at least one of the one or more organic layers includes the compound represented by Chemical Formula 1.

In such a case, the organic layer including the compound represented by Chemical Formula 1 may be selected from the group consisting of a light emitting layer, a light emitting auxiliary layer, a hole injection layer, a hole transport layer, an electron injection layer, a lifespan improvement layer, an electron transport layer, and an electron transport auxiliary layer. In this case, the compound represented by Chemical Formula 1 may be included as at least one material selected from the group consisting of a phosphorescent host material of the light emitting layer, an electron transport layer, and an electron transport auxiliary layer.

### EFFECTS OF THE INVENTION

According to an embodiment of the present invention, a compound represented by Chemical Formula 1 has excellent characteristics such as an electron transport ability, luminescence ability, heat resistance, and the like, and thereby is applicable as an organic layer material of an organic electroluminescent device.

In particular, when the compound represented by Chemical Formula 1 of the present invention is used as an electron transport layer material, an electron transport auxiliary layer material, or a light emitting auxiliary layer material, it may exhibit high thermal stability, low driving voltage, rapid mobility, high current efficiency and long lifespan characteristics compared to conventional host materials or electron transport materials.

Accordingly, the organic electroluminescent device including the compound of Chemical Formula 1 may be significantly improved in aspects such as excellent light emitting performance, low driving voltage, long lifespan, and high efficiency, and thus may be effectively applied to full color display panels and the like.

Effects according to the present invention are not limited by the description exemplified above, and more diverse effects are included in the present specification.

### DETAILED DESCRIPTION OF THE INVENTION

Hereinafter, the present invention will be described in detail.

### <Novel organic compound>

The present invention is to provide a novel compound having excellent thermal stability, carrier transport properties, and light-emitting properties.

According to the present invention, the compound represented by Chemical Formula 1 comprises, at both ends of the molecule, a fluorene-based moiety (core) having excellent conductivity and an azine-based moiety (e.g., a pyrimidine or triazine group) having superior electron withdrawing group (EWG) characteristics, which are connected through separate linkers (e.g., L₁ to L₂) to form a basic backbone structure, wherein a novel structural feature is characterized in that a phenyl group is introduced at a specific position of one benzene ring of the fluorene moiety that is not bonded to the azine-based moiety (EWG) among the two benzene rings of the fluorene moiety, specifically at the 5-position of the fluorene-based moiety.

Specifically, in a typical organic electroluminescent device, particularly a device employing a blue fluorescent emission layer, excitons must be generated in as large a quantity as possible within a narrow region in order to improve efficiency. In phosphorescent devices, when excitons are highly concentrated, device efficiency may be reduced due to triplet-triplet annihilation (TTA). In fluorescent devices, however, since the TTA phenomenon contributes to the generation of singlet (S₁) excitons, rapid electron transport and injection capability in the electron transport layer are required in order to effectively utilize the triplet (T₁) energy within the light emitting layer (e.g., emission layer, emissive layer).

To this end, in the present invention, a fluorene moiety is included as an essential component of the compound backbone structure, and a phenyl group is introduced at the 5-position of the fluorene moiety to increase the aromaticity of the molecule, thereby enhancing charge transport and injection capabilities.

More specifically, when a phenyl group is introduced at the 5-position of the fluorene-based moiety, the aromaticity of the molecule is increased compared to a compound without such a phenyl group, thereby promoting intermolecular overlap and increasing electron mobility in an organic layer including such compounds. That is, a larger amount of electrons can be injected into the emission layer, thereby contributing to the formation of more excitons in the emission layer, and consequently improving the efficiency of the organic electroluminescent device. In contrast, when a phenyl group is introduced at a position other than the 5-position of the fluorene-based moiety, the shape of the electron cloud within the molecule may be affected or the overall molecular structure may become tilted. As a result, the electron transport ability is inferior to that of a structure in which a phenyl group is introduced at the 5-position or a structure without phenyl substitution, which may increase the driving voltage of the device and further reduce device efficiency. In addition, whereas conventional fluorene-based compounds generally have a low glass transition temperature (Tg), the compound according to the present invention has a higher glass transition temperature (Tg) due to the introduction of a phenyl group at the 5-position of the fluorene moiety, thereby improving the high-temperature reliability of the panel.

Further, the compound represented by Chemical Formula 1 has a high triplet energy and thus can prevent excitons generated from an emissive layer from diffusing (moving) into an electron transport layer or a hole transport layer adjacent to the emissive layer. Therefore, the number of excitons contributing to light emission in the emissive layer is increased to improve the luminous efficiency of the device and enhance the durability and stability of the device, thereby efficiently increasing the lifespan of the device. Most of the developed materials enable low-voltage driving, thereby exhibiting physical properties, such as increased lifespan.

Furthermore, the compound represented by Chemical Formula 1 is highly advantageous for electron transport and exhibits low driving voltage, high efficiency, and long device lifetime characteristics. The excellent electron-transport capability of the compound enables high efficiency and fast mobility in an organic electroluminescent device, and allows for facile control of HOMO and LUMO energy levels depending on the orientation or position of substituents. Accordingly, an organic electroluminescent device employing the compound may exhibit superior electron-transport properties.

As described above, when the compound represented by Chemical Formula 1 of the present invention may be applicable as an organic layer material of an organic electroluminescent device, preferably a light emitting layer material (a blue, a green and/or a red phosphorescent host material), an electron transport/injection layer material, a hole transport/injection layer material, a light emitting auxiliary layer material, an electron transport auxiliary layer, and/or a lifespan enhancement layer material, the performance and lifetime characteristics of the organic electroluminescent device may be significantly improved. Accordingly, such an organic electroluminescent device can ultimately maximize the performance of a full color organic electroluminescent panel.

According to the present invention, the compound represented by Chemical Formula 1 comprises a nitrogen-containing heteroaromatic ring (e.g., an azine, such as a X₁ to X₃-containing ring) having electron-withdrawing group (EWG) characteristics with excellent electron transport ability, and a fluorene-based moiety substituted with a phenyl group at the 5-position so as to provide high conductivity and increase the aromaticity of the molecule, and these are bonded through a separate linker (e.g., L₁ to L₂) to form a basic skeleton structure.
at least one cyano group as electron withdrawing groups (EWG) with excellent electron transport ability, and they are bonded through an azine-based linker having another EWG characteristics (e.g., X₄ to X₆-containing ring) to form a basic skeleton structure.

In Chemical Formula 1 according to the present invention, the nitrogen-containing heterocycle (e.g., X₁ to X₃-containing ring) may be a monocyclic or polycyclic heteroaryl group (e.g., azine) containing at least two nitrogen atoms. For an example of the nitrogen-containing heteroaromatic ring (e.g., X₁ to X₃-containing ring), X₁ to X₃ may be the same as or different from each other, and may each independently be N or C(R₅), provided that at least two of X₁ to X₃ are N. For a specific example, X₁ to X₃ includes two to three nitrogen atoms (N). As such, since the heterocycle containing two to three nitrogen atoms (N) is included, more excellent electron absorption characteristics may be exhibited, which is advantageous for electron injection and transport.

Herein, R₅ may be selected from the group consisting of a hydrogen, a deuterium (D), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or combine with an adjacent group (e.g., R₅, Ar₁~Ar₂) to form a fused ring. In this case, when R₅ may be plural in number, the plurality of R₅ may be the same as or different from each other. Specifically, R₅ may preferably be selected from: a hydrogen, a deuterium (D), a C₁ to C₄₀ alkyl group, a C₆ to C₆₀ aryl group, and a heteroaryl group having 5 to 60 nuclear atoms.

In an embodiment of the present invention, the nitrogen-containing heteroaromatic ring (e.g., X₁ to X₃-containing ring) may be any one selected from the group consisting of the following structural formulas. However, it is not limited thereto. 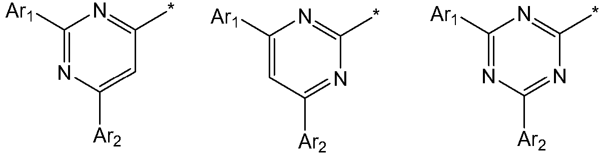 wherein the formulas,
* indicates a site where a bond with Chemical Formula 1 is made, and
Ar₁ and Ar₂ are each as defined in Chemical Formula 1.
the nitrogen-containing heterocycle (e.g., X₁ to X₃-containing ring) according to the present invention may each be substituted with Ar₁ to Ar₂ as various substituents. Such Ar₁ and Ar₂ may be the same as or different from each other and may each independently be selected from: a hydrogen, a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group. specifically, Ar₁ and Ar₂ may be each independently selected from the group consisting of a C₆ to C₆₀ aryl group, and a heteroaryl group having 5 to 60 nuclear atoms, more specifically, Ar₁ and Ar₂ may be each independently selected from the group consisting of a C₆ to C₄₀ aryl group, and a heteroaryl group having 5 to 60 nuclear atoms.

In an embodiment of the present invention, Ar₁ and Ar₂ may be the same as or different from each other and may each independently be more specifically embodied as any one of the following structural formulas. However, it is not limited thereto. 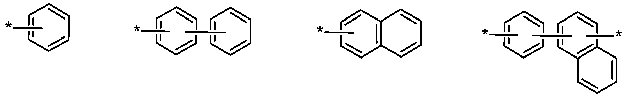 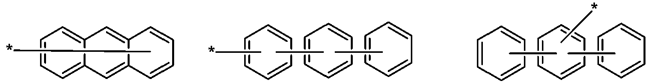 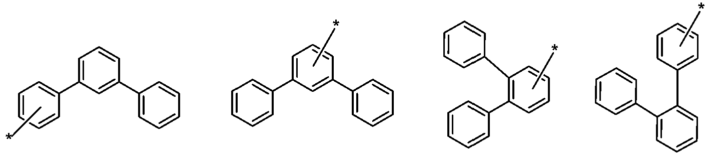 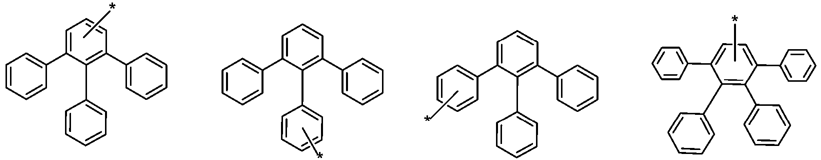 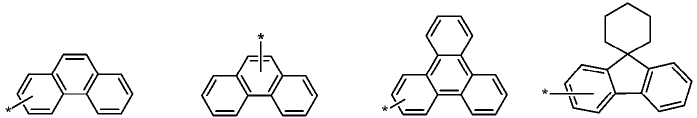 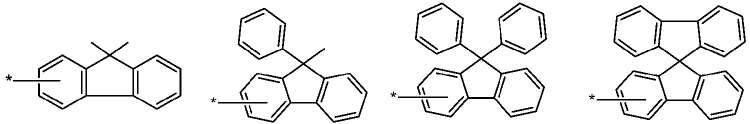 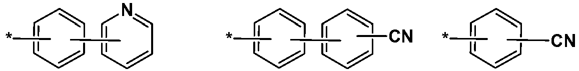 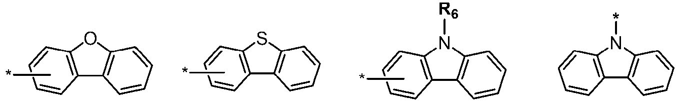 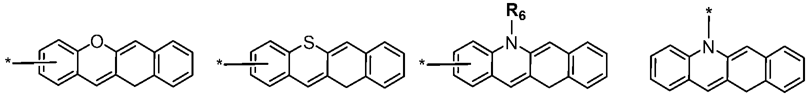 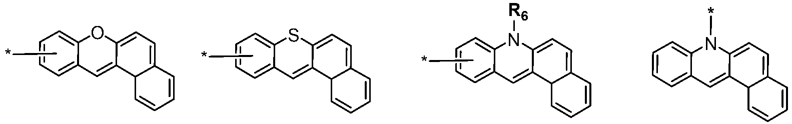 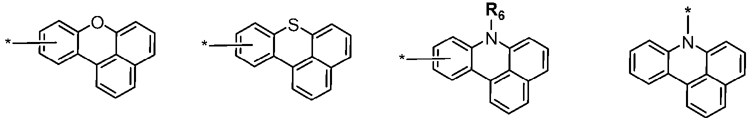 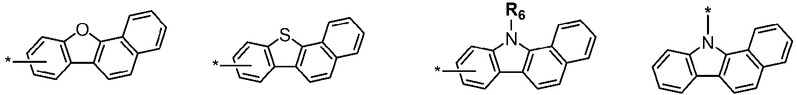 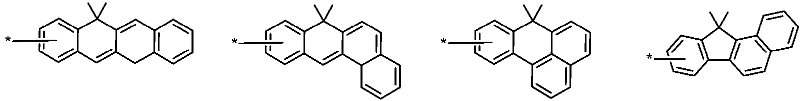 wherein the formulas,
* indicates a site where a bond with Chemical Formula 1 is made, and
R₆ may be selected from the group consisting of a hydrogen, a deuterium (D), a C₁ to C₄₀ alkyl group, a C₆ to C₆₀ aryl group, and a heteroaryl group having 5 to 60 nuclear atoms. In addition, although not shown in the above structural formulas, at least one substituent known in the art (e.g., the same as the definition of R₅) may be substituted.

In the compound represented by Chemical Formula 1 according to the present invention, a fluorene-based moiety is disposed on the opposite side of a nitrogen-containing heterocyclic ring (e.g., an X₁ to X₃-containing ring) with respect to the major axis of the molecule. Among the two benzene rings of the fluorene moiety, a phenyl group is introduced at a specific position of the benzene ring that is not bonded to the nitrogen-containing heteroaromatic ring, that is, at the 5-position of the fluorene-based moiety. The fluorene-based moiety having a phenyl group introduced at such a specific position increases the aromaticity of the molecule compared to a fluorene-based moiety without a phenyl group or having a phenyl group introduced at a position other than the 5-position, thereby improving charge transport ability and injection ability, as well as increasing the molecular weight and securing a high glass transition temperature (Tg) to enhance thermal stability. On the other hand, when a phenyl group is introduced at a position other than the 5-position of the fluorene-based moiety, the shape of the electron cloud formed within the molecule may be affected or the overall molecular structure may be tilted. Accordingly, the electron transport ability is inferior to that of the structure in which the phenyl group is introduced at the 5-position, which may result in an increase in driving voltage and/or a decrease in efficiency of the device.

R₁ to R₂ included in the fluorene-based moiety may be the same as or different from each other, and may each independently a hydrogen, a deuterium (D), a C₁ to C₄₀ alkyl group, or a C₆ to C₆₀ aryl group, or may be bonded to an adjacent group to form a condensed or fused ring having a monocyclic or polycyclic structure. The fluorene-derived fused ring may be a monocyclic or polycyclic alicyclic ring, a monocyclic or polycyclic heteroalicyclic ring, a monocyclic or polycyclic aromatic ring, or a monocyclic or polycyclic heteroaromatic ring, and for example, may be a monocyclic or polycyclic aromatic ring having 6 to 18 carbon atoms or a monocyclic or polycyclic heteroaromatic ring having 5 to 18 nuclear atoms. Specifically, R₁ and R₂ may each preferably be independently selected from the group consisting of a C₁ to C₂₀ alkyl group and a C₆ to C₄₀ aryl group or bonded to an adjacent group to form a fused ring.

In an embodiment of the present invention, the fluorene-based moiety may be any one selected from the following structural formulas. However, it is not limited thereto. 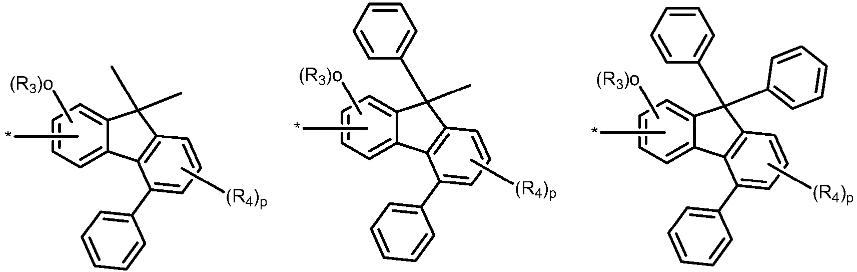 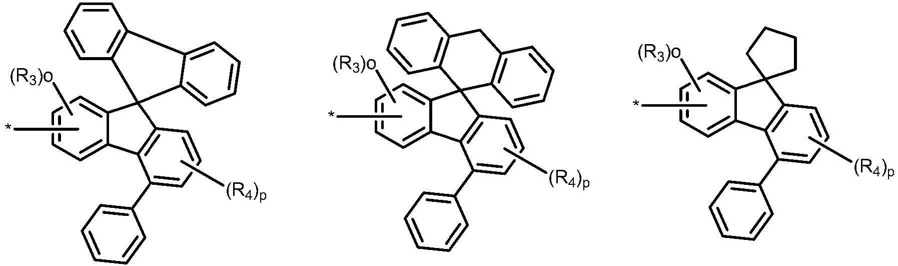 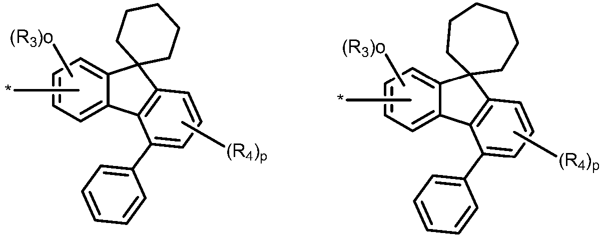 wherein the formulas,
* indicates a site where a bond with Chemical Formula 1 is made, and
R₃, R₄, o and p are each as defined in Chemical Formula 1. In addition, although not shown in the above structural formulas, at least one substituent known in the art (e.g., the same as the definition of R₅) may be substituted.

The fluorene-based moiety according to the present invention may each be substituted with R₃ to R₄ as various substituents. Such R₃ to R₄ may be the same as or different from each other, and may each independently a hydrogen, a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group, or may be bonded to any adjacent group to form a fused ring. In this case, when R₃ and R₄ may be plural in number, the plurality of R₃ and R₄ may be the same as or different from each other. Specifically, R₃ and R₄ may each preferably be independently selected from the group consisting of a hydrogen, a deuterium (D), a C₁ to C₂₀ alkyl group and a C₆ to C₄₀ aryl group and a heteroaryl group having 5 to 60 nuclear atoms.

Herein, o and p are each independently an integer of 0 to 3. When o is 0, R₃ is hydrogen, and when o is 1 to 3, R₃ may have the above-described substituents other than hydrogen. The same applies to p and R₄.

In the compound represented by Chemical Formula 1 according to the present invention, a nitrogen-containing heterocycle (e.g., X₁ to X₃) and the fluorene-based moiety are directly bonded to each other or connected through a separate linker (e.g., L₁ to L₂). As such, when the linker (e.g., L₁ to L₂) is present, a HOMO region may be expanded to improve a HOMO-LUMO distribution, and charge transfer efficiency may be enhanced through an appropriate overlap between the HOMO-LUMO.

L₁ and L₂ are not particularly limited, and may be a conventional divalent linking group known in the art. Specifically, L₁ and L₂ may be the same as or different from one another and may be each independently a single bond (a direct bond), or selected from the group consisting of a C₆ to C₁₈ arylene group and a heteroarylene group having 5 to 18 nuclear atoms. More specifically, L₁ and L₂ may each independently be selected from the group consisting of a C₆ to C₁₂ arylene group and a heteroarylene group having 5 to 12 nuclear atoms.

In this case, the numbers of linkers L₁ and L₂, that is, m and n, are each independently an integer from 0 to 2, provided that n + m ≥ 1. For example, when n is 0, this corresponds to a single bond (direct bond), and thus the single bond is excluded from L₁ and L₂. In addition, when m and n are each 1 to 2, a plurality of L₁ and L₂ groups may be the same as or different from each other, and may each independently be selected from the abovedefined linker groups except for a single bond.

Specific examples of the arylene linker and the heteroarylene linker include a phenylene group, a biphenylene group, a naphthylene group, an anthracenylene group, an indenylene group, a phenanthrenylene group, a carbazolylene group, a thiophenylene group, an indolylene group, a furanylene group, a quinolinylene group, a pyrrolylene group, an imidazolylene group, an oxazolylene group, a thiazolylene group, a pyridinylene group, a pyrimidinylene group, a dibenzofuranylene group, a dibenzothiophenylene group, and/or a dibenzoselenophenylene group. More specifically, the arylene linker is preferably a phenylene group or a biphenylene group.

In an embodiment of the present invention, L₁ and L₂ may be the same as or different from each other, and may each independently be selected from the group consisting of the following structural formulas. However, it is not limited thereto. 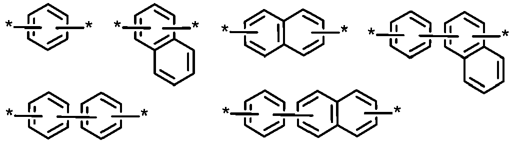 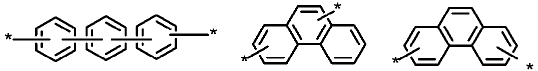 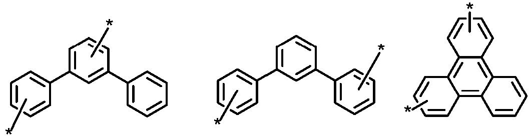 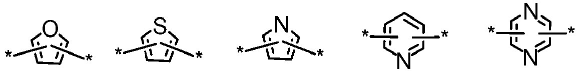 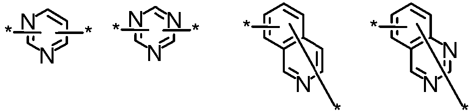 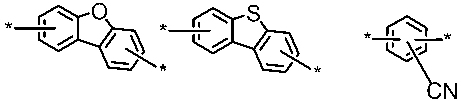 wherein the formulas,
* indicates a site where a bond with Chemical Formula 1 is made. In addition, although not shown in the above structural formulas, at least one substituent known in the art (e.g., the same as the definition of R₅) may be substituted.

In the above Chemical Formula 1, the arylene group, the heteroarylene group of L₁ and L₂; and the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, and arylamine group of Ar₁ to Ar₂, and R₁ to R₃ may be each independently substituted with at least one substituent selected from the group consisting of a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and wherein when the substituent is present in a plural number, they may be the same or different from each other.

In an embodiment of the present invention, the compound represented by Chemical Formula 1 may be more specifically represented by the following Chemical Formula 2 or Chemical Formula 3, depending on whether R₁ and R₂ included in the fluorene-based moiety form a ring. 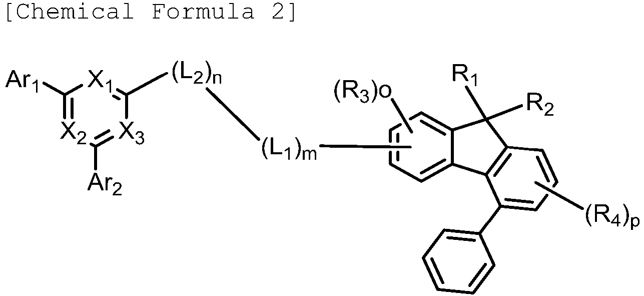 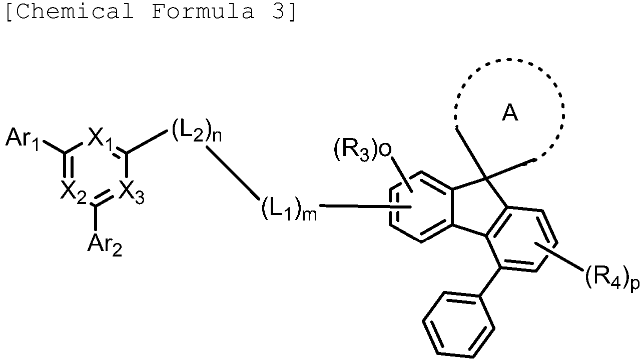 wherein the formulas,
Ring A may be a monocyclic or polycyclic hydrocarbon ring having 5 to 18 carbon atoms, which may or may not include a heteroatom. Such Ring A may be a condensed or fused monocyclic or polycyclic hydrocarbon ring or a nitrogen-containing ring known in the art, and for example, may be an alicyclic ring, a heteroalicyclic ring, an aromatic ring, or a heteroaromatic ring. Preferably, Ring A may be an aromatic ring having 6 to 18 carbon atoms or a polycyclic heteroaromatic ring having 5 to 12 nuclear atoms and including a heteroatom. Herein, the heteroatom may be N, O, or S
X₁ to X₃, Ar₁ to Ar₂, L₁ to L₂, R₁ to R₄, n, m, o and p are each as defined in Chemical Formula 1, provided that cases where R₁ and R₂ are simultaneously hydrogen are excluded.

In another embodiment of the present invention, the compound represented by Chemical Formula 1 may be further embodied as any one of the following Chemical Formulas 4 to 7 depending on the bonding position between the linker (e.g., L₁) and the fluorene-based moiety. However, it is not limited thereto. 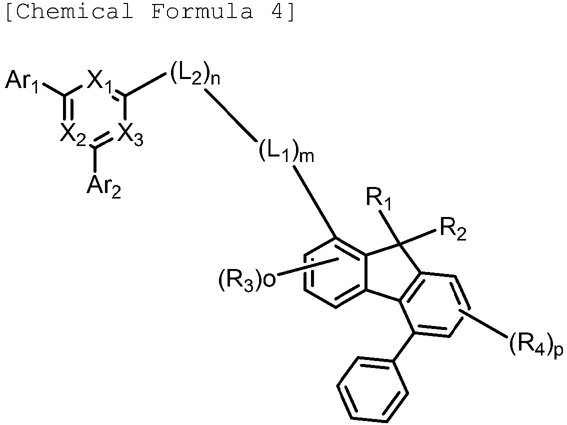 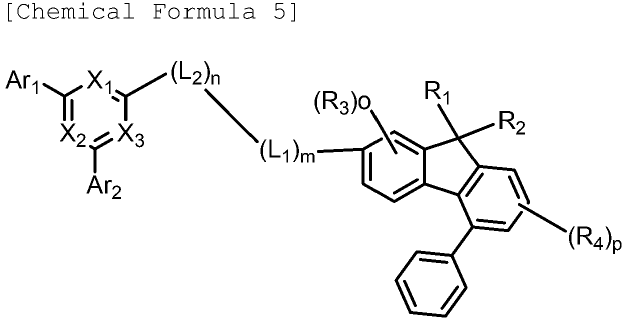 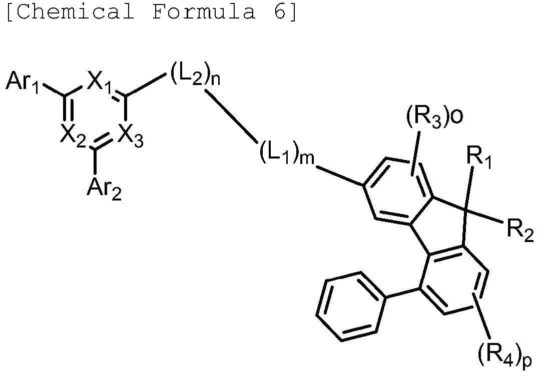 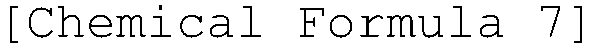 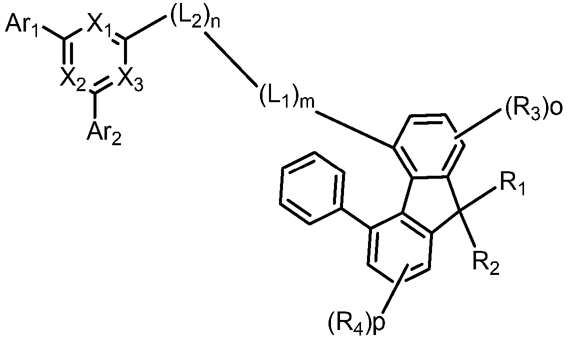 wherein the formulas,
X₁ to X₃, Ar₁ to Ar₂, L₁ to L₂, R₁ to R₄, n, m, o and p are each as defined in Chemical Formula 1.

In another embodiment of the present invention, the compound represented by Chemical Formula 1 may be further embodied as any one of the following Chemical Formulas 8 to 15 depending on the type of the fluorene-based moiety. However, it is not limited thereto. 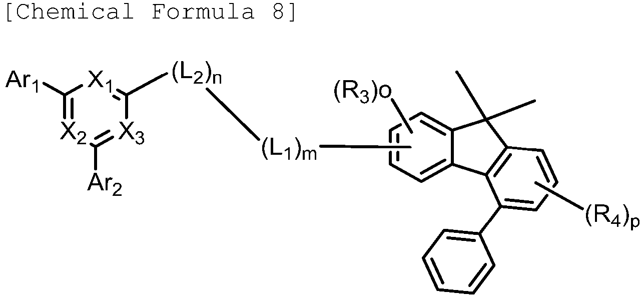 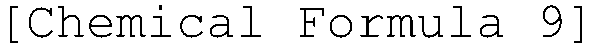 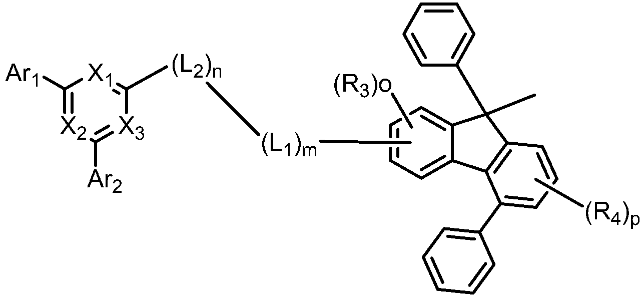 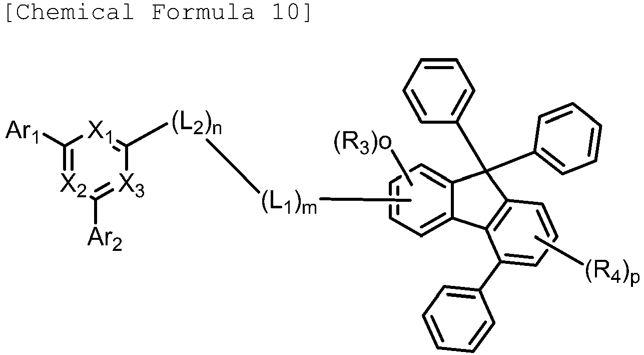 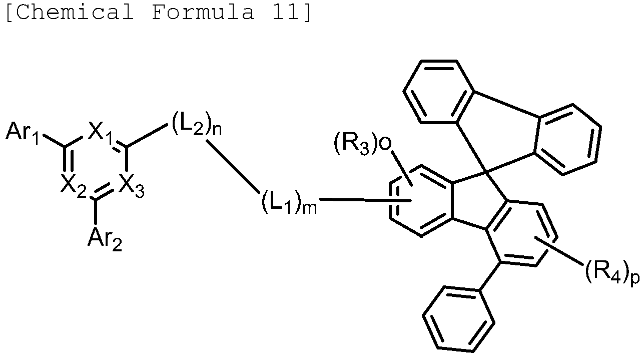 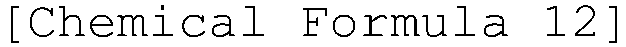 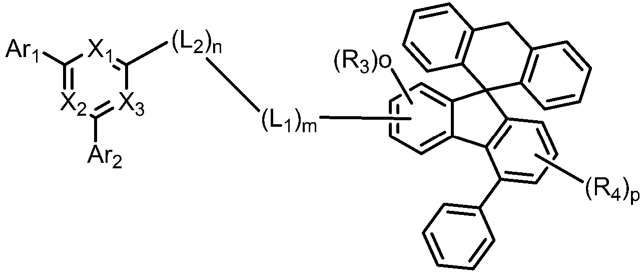 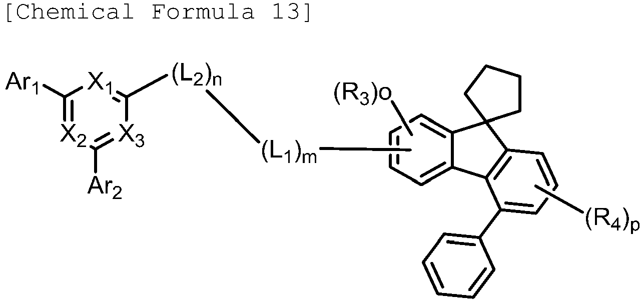 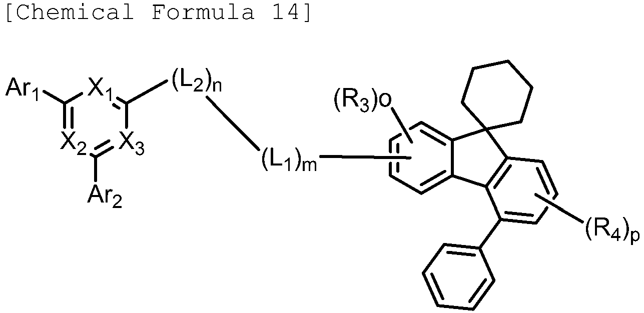 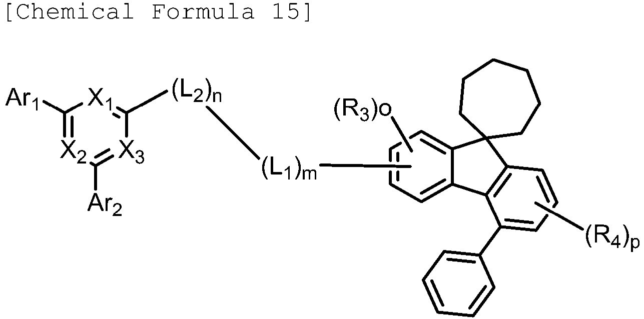 wherein the formulas,
X₁ to X₃, Ar₁ to Ar₂, L₁ to L₂, R₃ to R₄, n, m, o and p are each as defined in Chemical Formula 1.

In another embodiment of the present invention, the compound represented by Chemical Formula 1 may be further embodied as any one of the following Chemical Formulas 16 to 18 depending on the type of the nitrogen-containing heterocycle (e.g., the ring containing X₁ to X₃). However, it is not limited thereto. 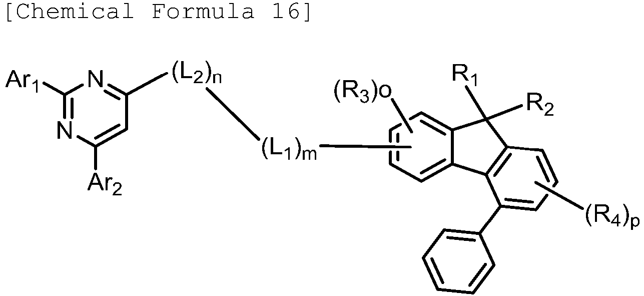 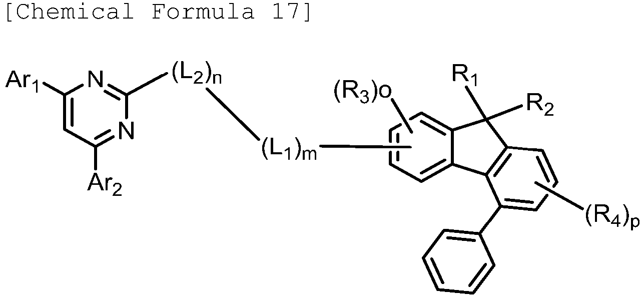 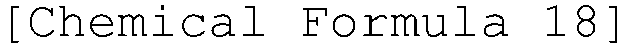 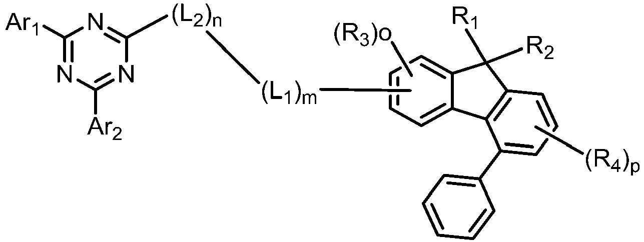 wherein the formulas,
Ar₁ to Ar₂, L₁ to L₂, R₁ to R₄, n, m, o and p are each as defined in Chemical Formula 1.

In another embodiment of the present invention, the compound represented by Chemical Formula 1 may be further embodied as any one of the following Chemical Formulas 19 to 23 depending on the type of the linkers (e.g., L₁ to L₂). However, it is not limited thereto. 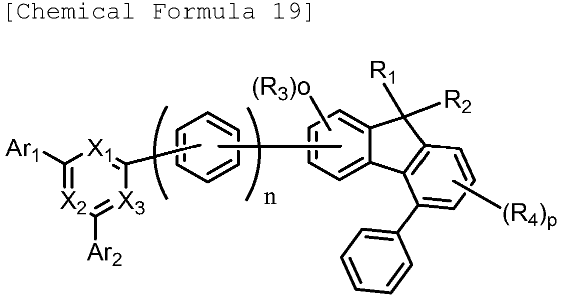 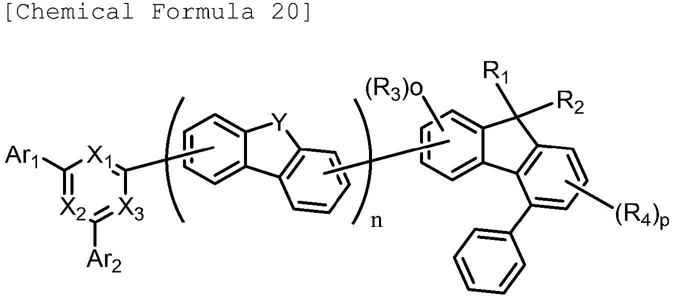 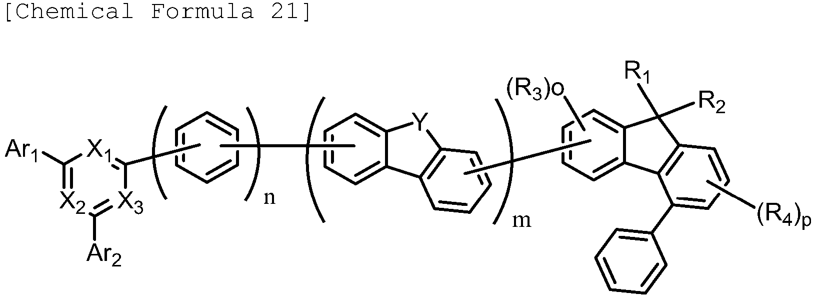 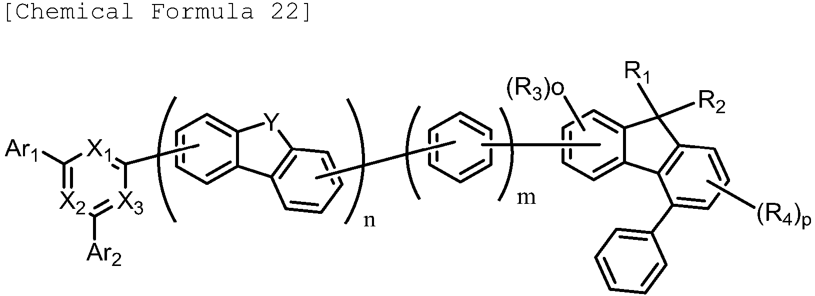 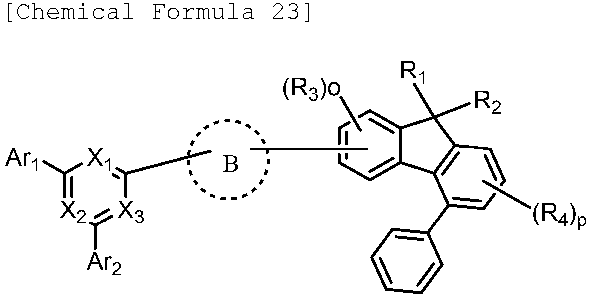 wherein the formulas,
Ring B may be a divalent fused polycyclic aromatic group having 10 to 30 carbon atoms (C₁₀ to C₃₀) commonly known in the art, and specifically, may be selected from the group consisting of a naphthalene group, a phenalene group, an anthracene group, a fluoranthene group, a triphenylene group, a phenanthrene group, a pyrene group, a chrysene group, and a perylene group;
Y is O or S,
X₁ to X₃, Ar₁ to Ar₂, R₁ to R₄, n, m, o and p are each as defined in Chemical Formula 1.

The compound represented by Chemical Formula 1 of the present invention described above may be further embodied as compounds represented by the following compounds represented by, for example, 1 to 208. However, the compound represented by Chemical Formula 1 of the present invention is not limited to those exemplified below.

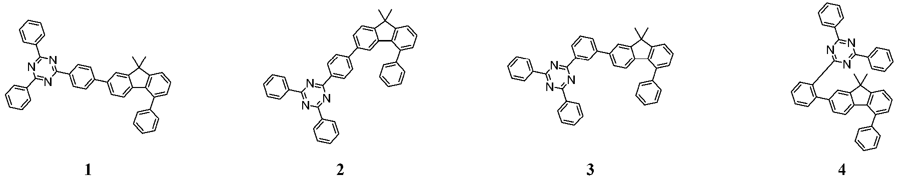

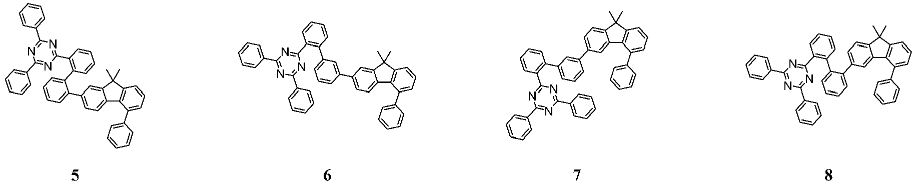

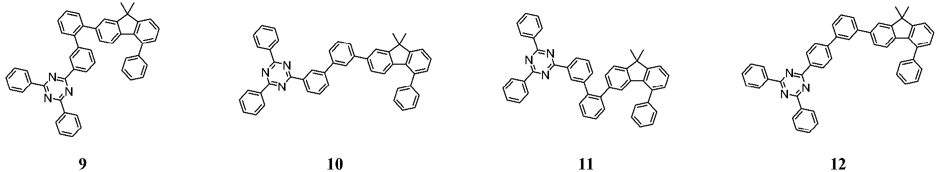

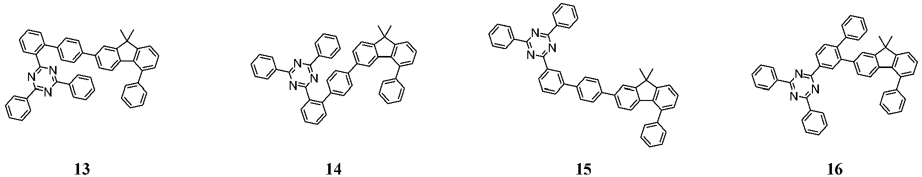

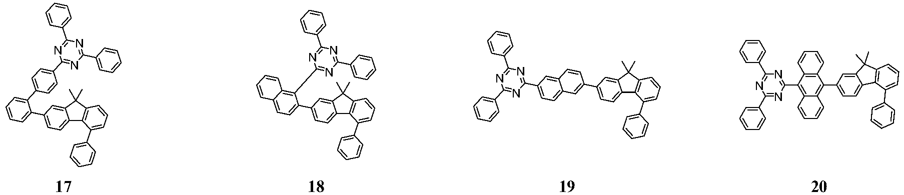

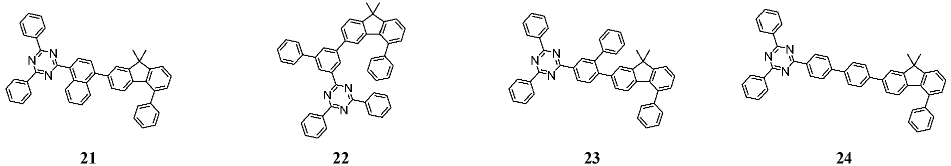

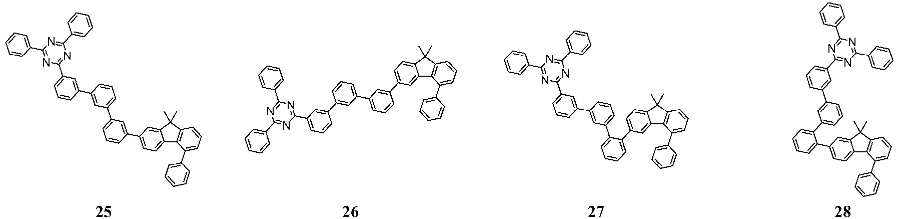

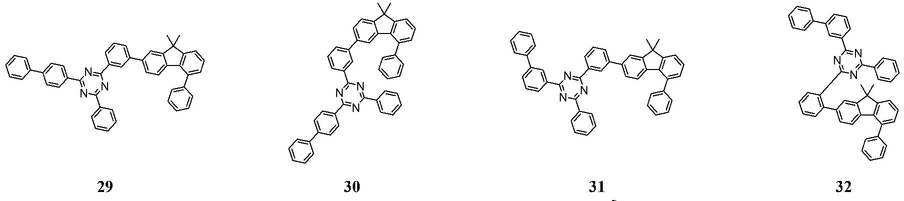

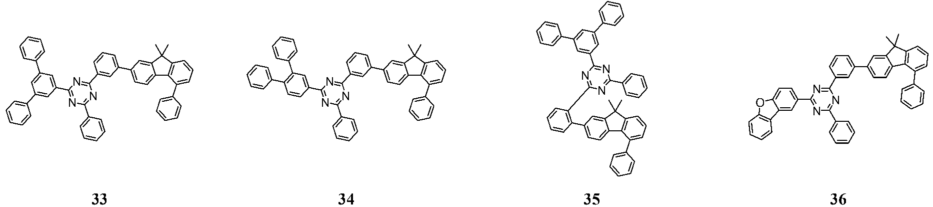

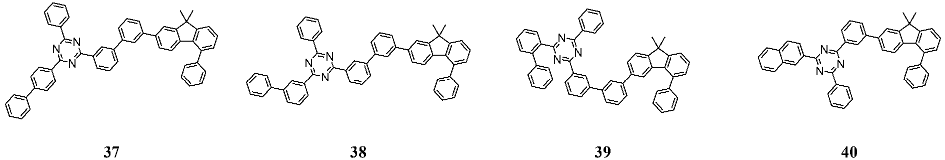

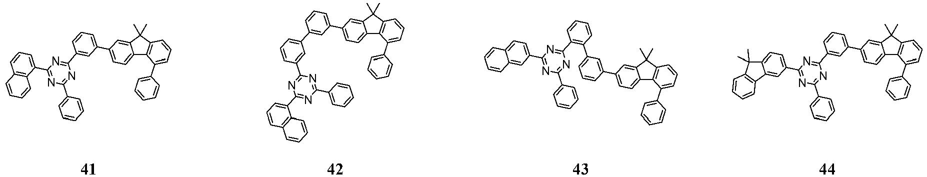

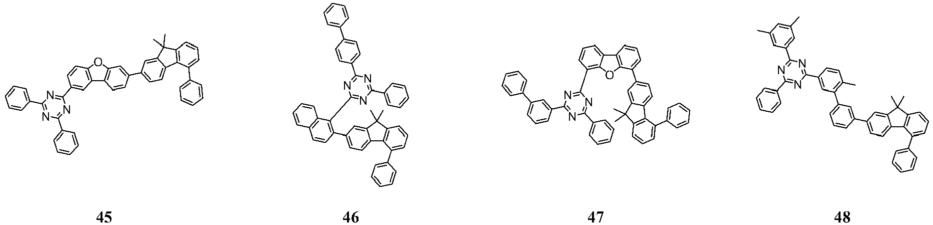

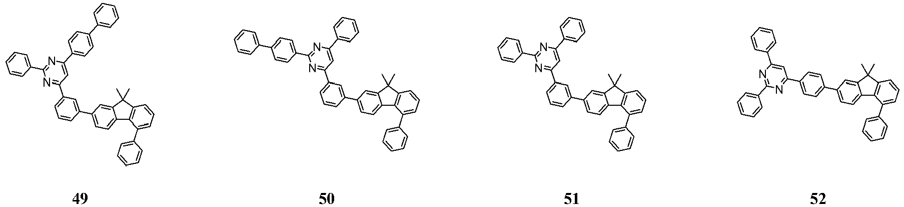

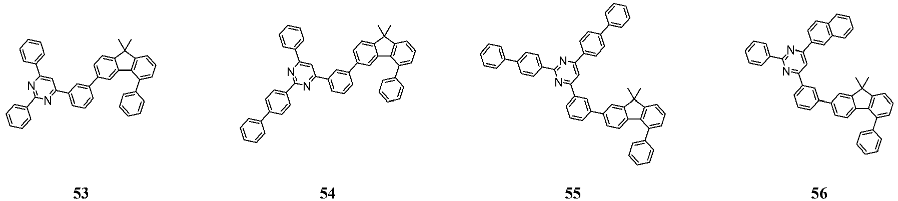

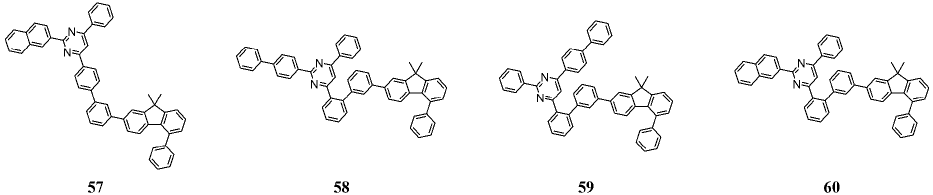

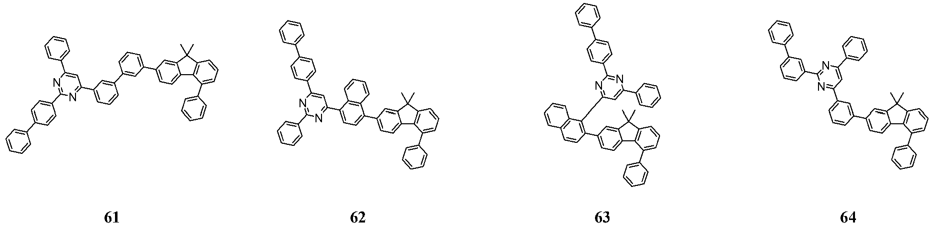

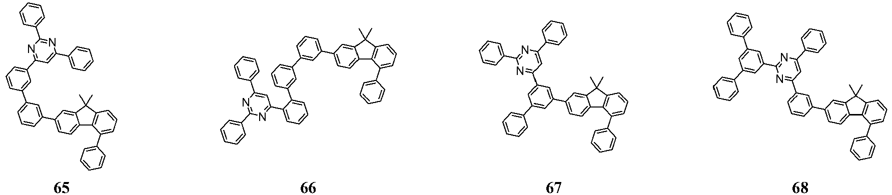

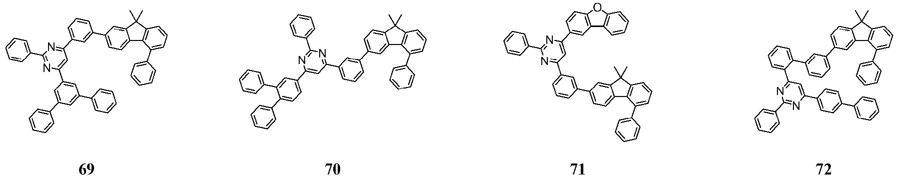

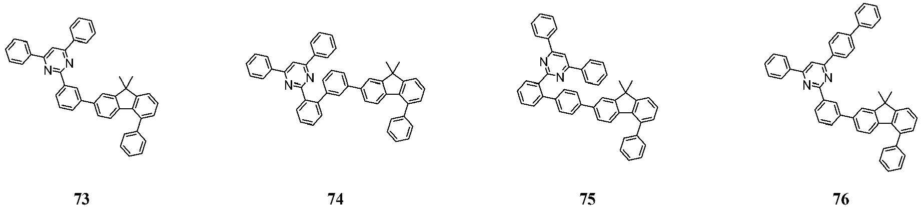

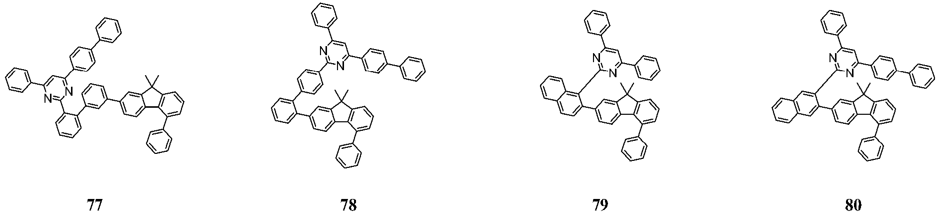

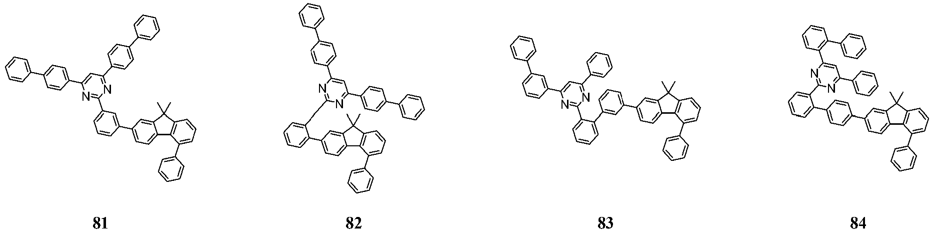

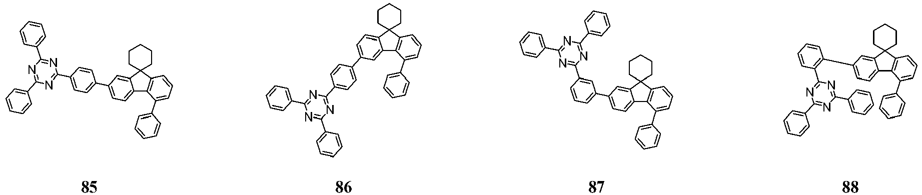

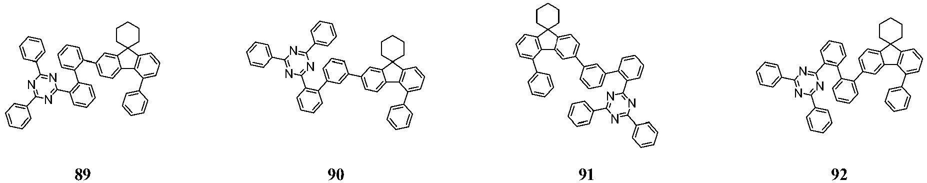

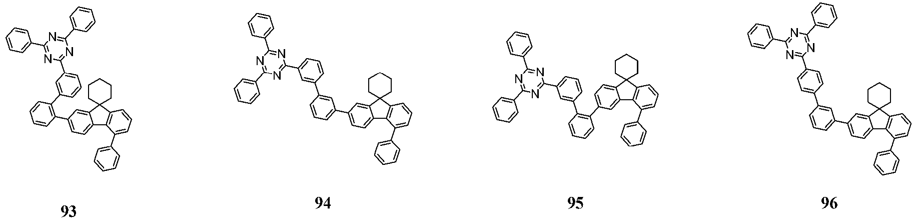

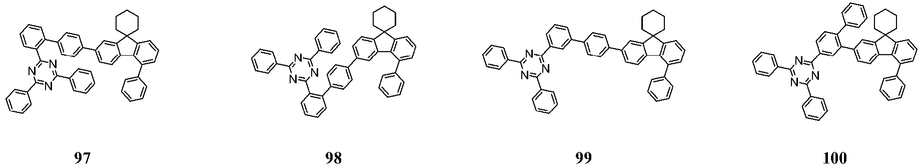

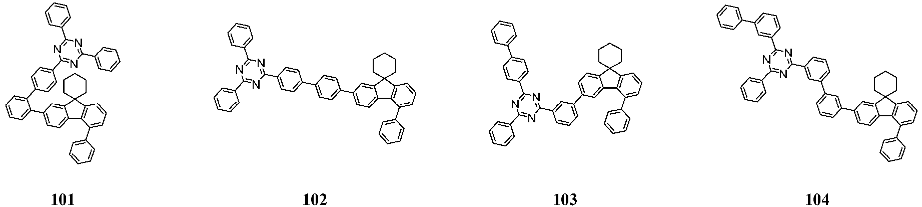

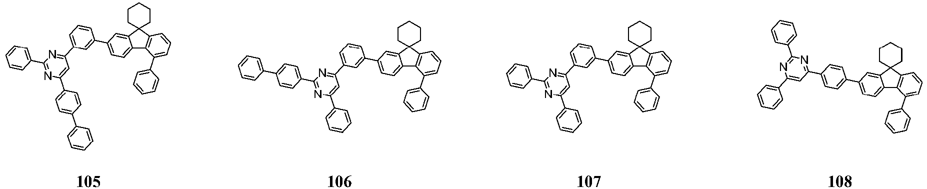

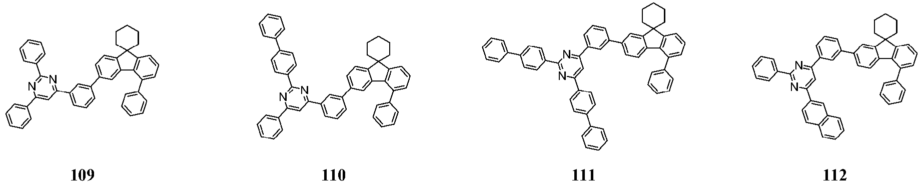

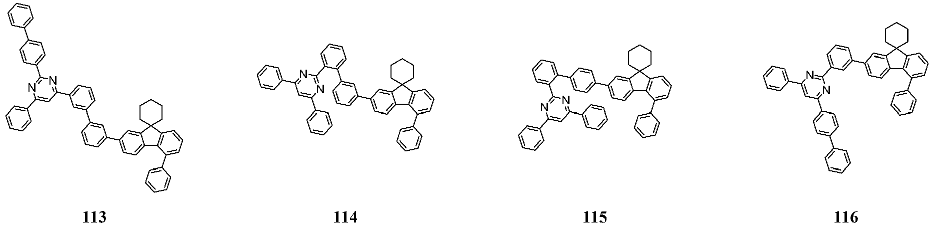

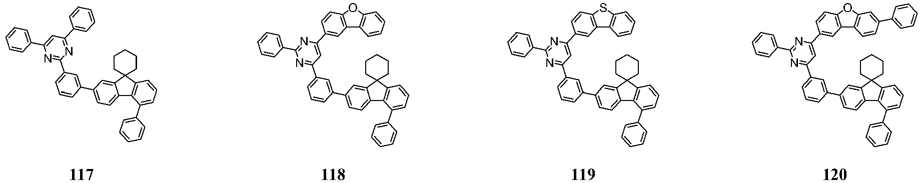

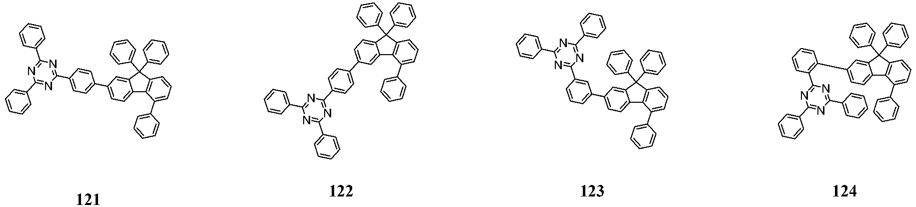

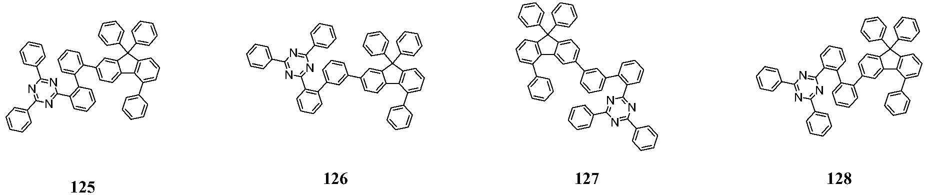

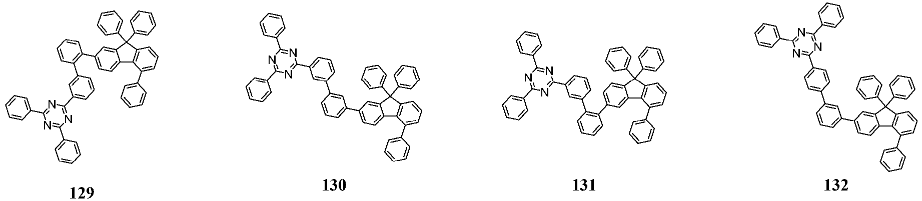

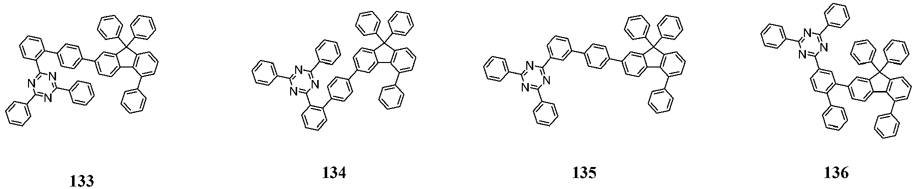

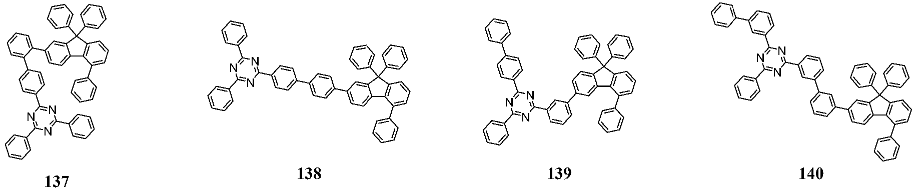

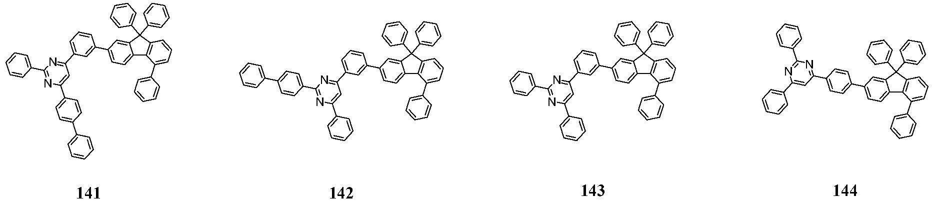

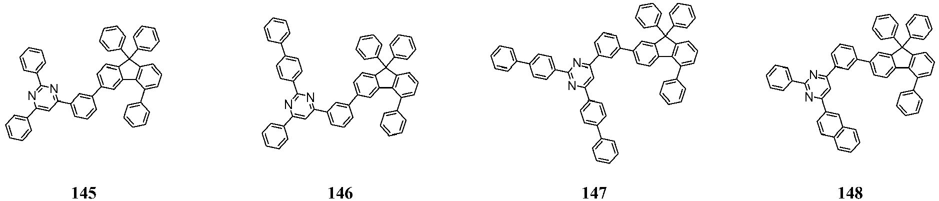

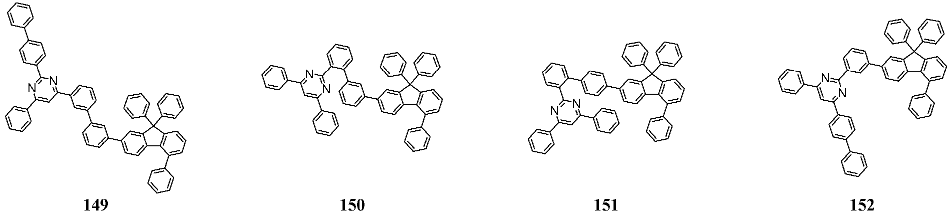

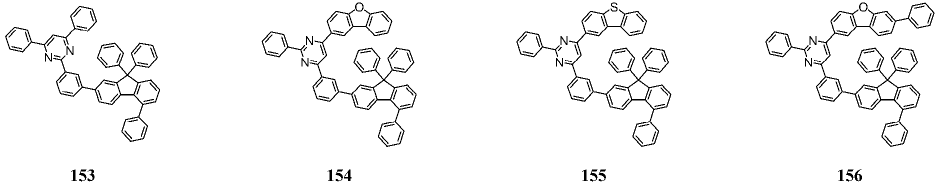

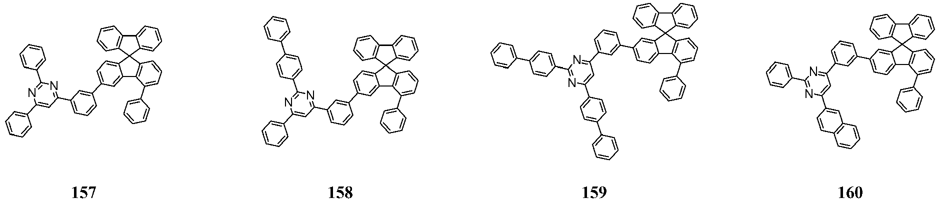

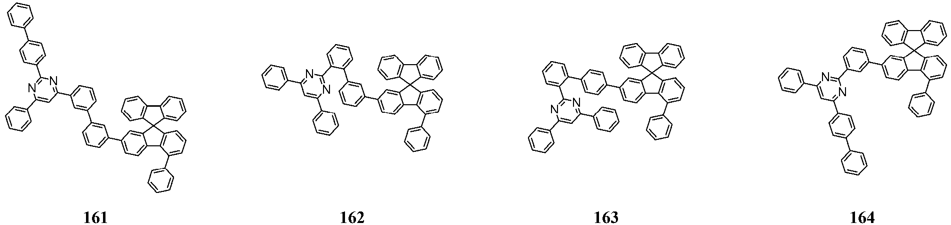

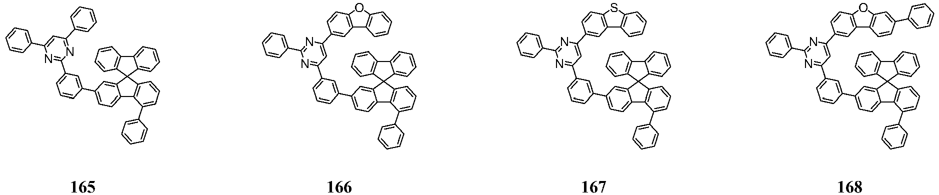

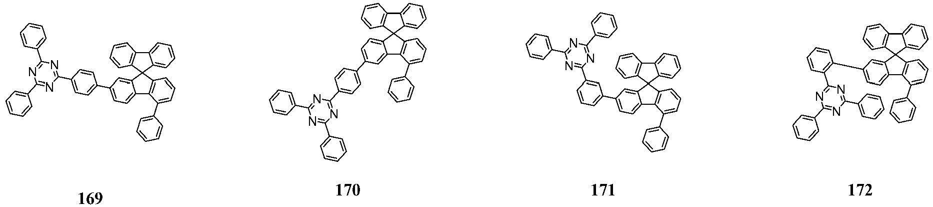

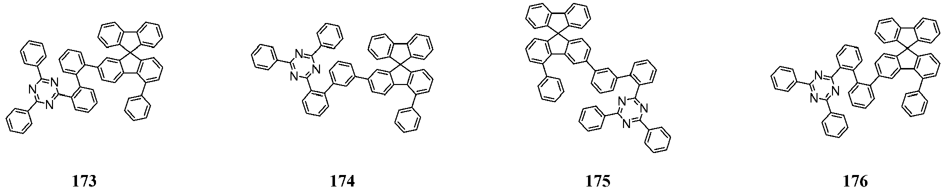

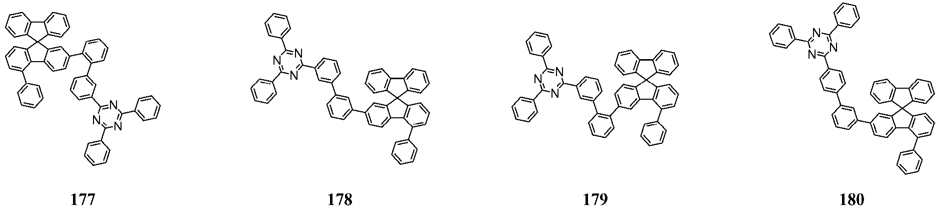

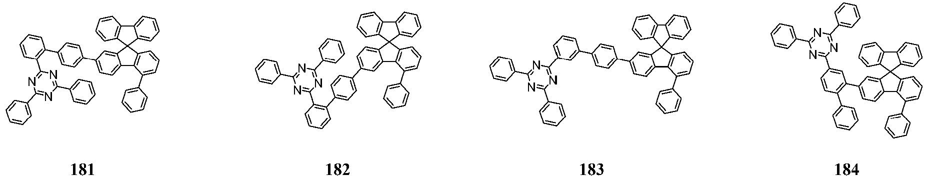

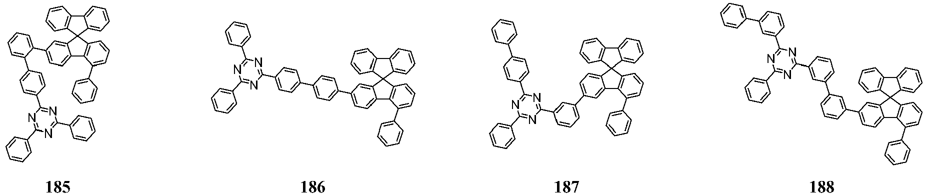

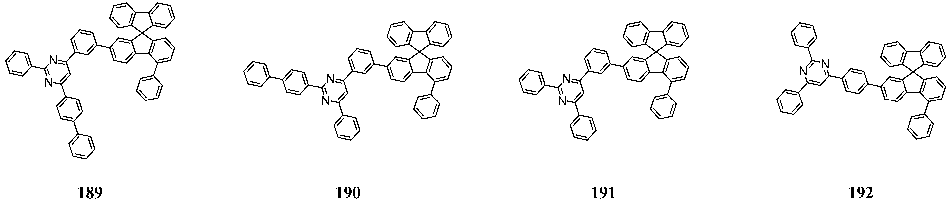

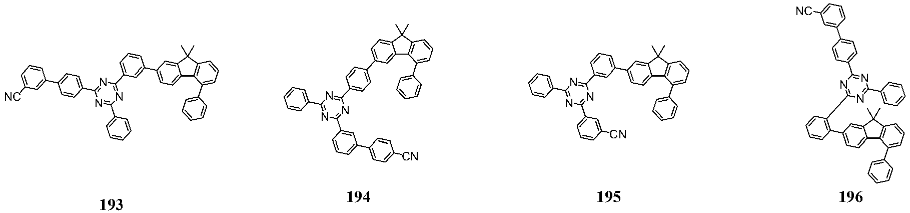

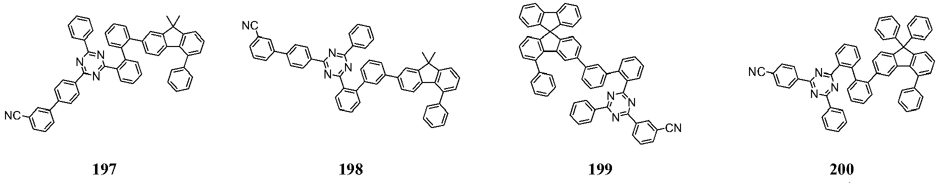

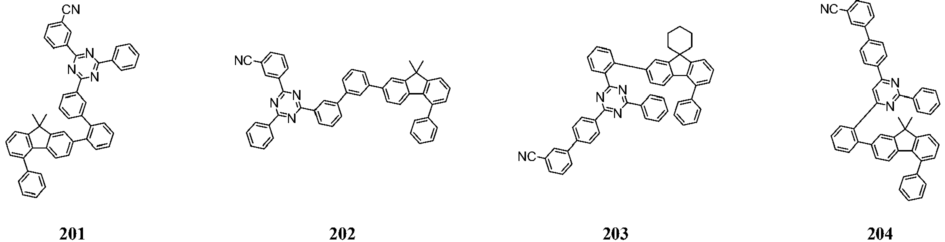

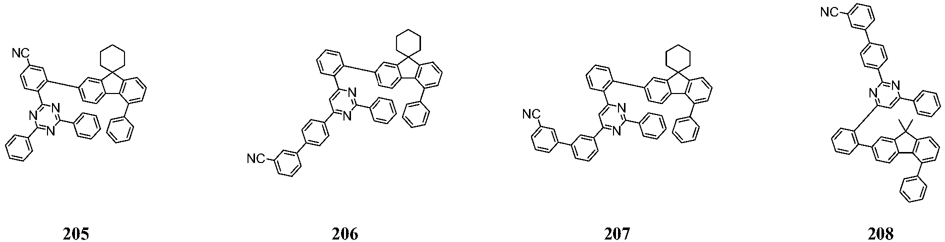

As used herein, "the number of nuclear atoms" means the number of nuclear atoms constituting a ring structure, such as ring atoms, and the nuclear atoms may mean carbon or a heteroatom selected from the group consisting of N, O, S and Se. For example, the number of nuclear atoms of pyridine means 6 including 5 C and 1 N constituting a pyridine ring.

As used herein, "alkyl" refers to a monovalent substituent derived from a linear or branched chain saturated hydrocarbon having 1 to 40 carbon atoms. Examples of such alkyl may include, but not limited to, methyl, ethyl, propyl, isobutyl, sec-butyl, pentyl, iso-amyl, hexyl or the like.

As used herein, "alkenyl" refers to a monovalent substituent derived from a C₂ to C₄₀ linear or branched chain unsaturated hydrocarbon, having at least one carbon-carbon double bond. Examples of such alkenyl may include, but not limited to, vinyl, allyl, isopropenyl, 2-butenyl or the like.

As used herein, "alkynyl" refers to a monovalent substituent derived from a C₂ to C₄₀ linear or branched chain unsaturated hydrocarbon, having at least one carbon-carbon triple bond. Examples of such alkynyl may include, but not limited to, ethynyl, 2-propynyl or the like.

As used herein, "aryl" refers to a monovalent substituent derived from a C₆ to C₄₀ aromatic hydrocarbon having a structure with a single ring or two or more rings combined with each other. In addition, a form in which two or more rings are pendant (e.g., simply attached) to or fused with each other may also be included. Examples of such aryl may include, but not limited to, phenyl, naphthyl, phenanthryl, anthryl or the like.

As used herein, "heteroaryl" refers to a monovalent substituent derived from a monoheterocyclic or polyheterocyclic aromatic hydrocarbon having 5 to 40 nuclear atoms. In such an embodiment, one or more carbons in the ring, preferably one to three carbons, are substituted with a heteroatom such as N, O, S or Se. In addition, a form in which two or more rings are pendant to or fused with each other may be included and a form fused with an aryl group may be included. Examples of such heteroaryl may include, but not limited to, a 6-membered monocyclic ring such as pyridyl, pyrazinyl, pyrimidinyl, pyridazinyl and triazinyl; a polycyclic ring such as phenoxathienyl, indolizinyl, indolyl, purinyl, quinolyl, benzothiazole, and carbazolyl; 2-furanyl; N-imidazolyl; 2-isoxazolyl; 2-pyridinyl; 2-pyrimidinyl or the like.

As used herein, "aryloxy" is a monovalent substituent represented by RO-, where R refers to a C₅ to C₄₀ aryl. Examples of such aryloxy may include, but not limited to, phenyloxy, naphthyloxy, diphenyloxy or the like.

As used herein, "alkyloxy" refers to a monovalent substituent represented by R'O-, where R' refers to a C₁ to C₄₀ alkyl. Such alkyloxy may include a linear, branched or cyclic structure. Examples of such alkyloxy may include, but not limited to, methoxy, ethoxy, n-propoxy, 1-propoxy, t-butoxy, n-butoxy, pentoxy or the like.

As used herein, "arylamine" refers to amine substituted with a C₆ to C₄₀ aryl.

As used herein, "cycloalkyl" refers to a monovalent substituent derived from a C₃ to C₄₀ monocyclic or polycyclic non-aromatic hydrocarbon. Examples of such cycloalkyl may include, but not limited to, cyclopropyl, cyclopentyl, cyclohexyl, norbornyl, adamantine or the like.

As used herein, "heterocycloalkyl" refers to a monovalent substituent derived from a non-aromatic hydrocarbon having 3 to 40 nuclear atoms, where one or more carbons in the ring, preferably one to three carbons, are substituted with a heteroatom such as N, O, S or Se. Examples of such heterocycloalkyl may include, but not limited to, morpholine, piperazine or the like.

As used herein, "alkylsilyl" refers to silyl substituted with a C₁ to C₄₀ alkyl, and "arylsilyl" refers to silyl substituted with a C₅ to C₄₀ aryl.

As used herein, the term "fused ring (e.g., condensed ring)" refers to a condensed aliphatic ring, a condensed aromatic ring, a condensed heteroaliphatic ring, a condensed heteroaromatic ring, or a combination thereof.

### <Electron transport layer material>

The present invention provides an electron transport layer including the compound represented by Chemical Formula 1.

The electron transport layer (ETL) serves to move electrons injected from a cathode to an adjacent layer, specifically a light emitting layer.

The compound represented by Chemical Formula 1 may be used alone as an electron transport layer (ETL) material, or may be used in combination with an electron transport layer material known in the art. It may preferably be used alone.

The electron transport layer material that may be used in combination with the compound of Chemical Formula 1 may include an electron transport material commonly known in the art. Nonlimiting examples of applicable electron transport materials may include oxazole-based compounds, isoxazole-based compounds, triazole-based compounds, isothiazole-based compounds, oxadiazole-based compounds, thiadiazole-based compounds, perylene-based compounds, aluminum complexes (e.g., tris(8-quinolinolato)-aluminium (Alq₃), BAlq, SAlq, Almq₃, gallium complexes (e.g., Gaq'2OPiv, Gaq'2OAc, 2(Gaq'2)), etc. These may be used alone or two or more types thereof may be used in combination.

In the present invention, when the compound of Chemical Formula 1 and the electron transport layer material are used in combination, a mixing ratio thereof is not particularly limited, and may be appropriately adjusted within a range known in the art.

### <an electron transport auxiliary layer material>

In addition, the present invention provides an auxiliary electron transport layer including the compound represented by Chemical Formula 1.

The auxiliary electron transport layer is disposed between the light emitting layer and the electron transport layer and serves to substantially prevent diffusion of excitons or holes generated in the light emitting layer into the electron transport layer.

The compound represented by Chemical Formula 1 may be used alone as an auxiliary electron transport layer material, or may be combined with an electron transport layer material known in the art. It may preferably be used alone.

The auxiliary electron transport layer material that may be used in combination with the compound of Chemical Formula 1 includes an electron transport material commonly known in the art. For example, the auxiliary electron transport layer may include an oxadiazole derivative, a triazole derivative, a phenanthroline derivative (e.g., BCP), a heterocyclic derivative containing nitrogen, and the like.

In the present invention, when the compound of Chemical Formula 1 and the auxiliary electron transport layer material are used in combination, a mixing ratio thereof is not particularly limited, and may be appropriately adjusted within a range known in the art.

### <Organic electroluminescent device>

Another aspect of the present invention is directed to an organic electroluminescent device ("organic EL device" or "organic EL element") including the compound represented by Chemical Formula 1.

More specifically, the organic EL device according to the present invention includes an anode (e.g., a positive electrode), a cathode (e.g., a negative electrode), and one or more organic layers disposed between the anode and the cathode, and at least one of the one or more organic layers includes the compound represented by Chemical Formula 1. In such an embodiment, the compound may be used alone or in combination of two or more kinds thereof.

The one or more organic layers may be any one or more of a hole injection layer, a hole transport layer, a light emitting layer, a light emitting auxiliary layer, a lifespan improvement layer, an electron transport layer, an electron transport auxiliary layer and an electron injection layer, and at least one of the organic layers may include the compound represented by Chemical Formula 1. Specifically, the organic layer including the compound represented by Chemical Formula 1 may preferably be a light emitting layer, a light emitting auxiliary layer, an electron transport layer, an electron transport auxiliary layer and/or a lifespan improvement layer, and more specifically, it may be preferable to be an electron transport layer, an electron transport auxiliary layer or a light emitting auxiliary layer.

The light emitting layer of the organic EL device of the present invention includes a host material and a dopant material, and may include the compound of Chemical Formula 1 as a host material. In addition, the light emitting layer of the present invention may include, as a host, other compounds known in the art rather than or in addition to the compound of Chemical Formula 1.

When the compound represented by Chemical Formula 1 is included as a material for the light emitting layer of the organic EL device, preferably as a blue, green, or red phosphorescent host material, since a bonding force between holes and electrons in the light emitting layer is increased, the efficiency (luminescence efficiency and power efficiency), lifespan, luminance, driving voltage, and the like of the organic EL device may be improved. Specifically, the compound represented by Chemical Formula 1 may be preferably included in an organic EL device as a green and/or red phosphorescent host, a fluorescent host, or a dopant material. In particular, the compound represented by Chemical Formula 1 of the present invention may preferably be a green phosphorescent exciplex N-type host material for a high-efficiency light emitting layer.

A structure of the organic EL device of the present invention is not particularly limited, and may be, for example, a structure in which a substrate, an anode, a hole injection layer, a hole transport layer, a light emitting auxiliary layer, a light emitting layer, an electron transport layer, and a cathode are sequentially stacked. In such a case, at least one of the hole injection layer, the hole transport layer, the light emitting auxiliary layer, the light emitting layer, the electron transport layer and the electron injection layer may include the compound represented by Chemical Formula 1, and preferably, the light emitting layer, more preferably a phosphorescent host, may include the compound represented by Chemical Formula 1. Meanwhile, an electron injection layer may be additionally stacked on the electron transport layer.

The organic EL device of the present invention may have a structure in which an insulating layer or an adhesive layer is inserted at an interface between the electrode and the organic layer.

The organic EL device of the present invention may be prepared using materials and methods known in the art to form organic layers and electrodes, except that one or more layers of the aforementioned organic layers include the compound represented by Chemical Formula 1.

The organic layer may be formed by a vacuum deposition method or a solution coating method. Examples of the solution coating method may include, but not limited to, spin coating, dip coating, doctor blading, inkjet printing, thermal transfer or the like.

The substrate used in preparation of the organic EL device of the present invention is not particularly limited, and nonlimiting examples thereof may include silicon wafers, quartz, glass plates, metal plates, plastic films, sheets or the like.

In addition, an anode material may use any anode material known in the art without limitation. Examples of the anode material may include, but not limited to, a metal such as vanadium, chromium, copper, zinc, and gold or an alloy thereof; metal oxides such as zinc oxide, indium oxide, indium tin oxide (ITO), or indium zinc oxide (IZO); combination of oxide with metal such as ZnO:Al or SnO₂:Sb; conductive polymers such as polythiophene, poly(3-methylthiophene), poly [3,4-(ethylene-1,2-dioxy) thiophene] (PEDT), polypyrrole or polyaniline; carbon black or the like.

In addition, a cathode material may use any cathode material known in the art without limitation. Examples of the cathode material may include, but not limited to, a metal such as magnesium, calcium, sodium, potassium, titanium, indium, yttrium, lithium, gadolinium, aluminum, silver, tin, or lead or an alloy thereof; a multi-layered material such as LiF/Al or LiO₂/Al or the like.

In addition, materials for the hole injection layer, the hole transport layer, the electron injection layer, and the electron transport layer are not particularly limited, and conventional materials known in the art may be used without limitation.

Hereinafter, the present invention will be described in detail through examples. However, the following examples are only to illustrate the present invention, and the present invention is not limited by the following examples.

### <Preparation Examples 1 to 4>

### [Preparation Example 1]

### Synthesis of Core 1

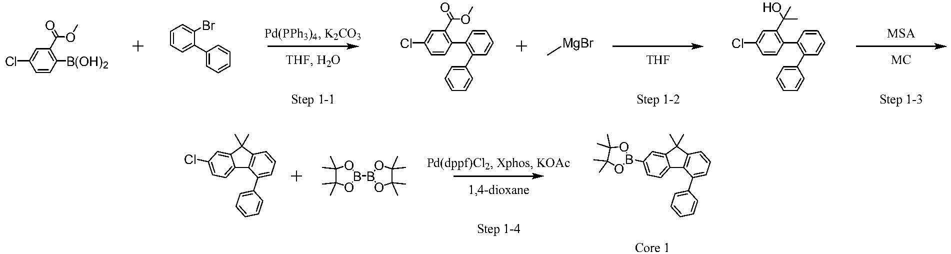

### <Step 1-1>

(4-chloro-2-(methoxycarbonyl)phenyl)boronic acid 100.0 g(466.4 mmol), 2-bromo-1,1'-biphenyl 119.6 g(513.0 mmol), Pd(PPh₃)₄ 23.3 g(26.9 mmol), K₂CO₃ 193.4 g (1399.2 mmol), 1000 ml of THF, and 250 ml of H₂O were added, and the mixture was stirred under reflux at 80°C for 8 hours. After completion of the reaction, the organic layer was extracted using H₂O and ethyl acetate, and then dried over MgSO₄ and filtered. The filtered organic layer was concentrated under reduced pressure, and the residue was recrystallized using MC and MeOH. The resulting solid was filtered, washed with MeOH, and dried in an oven to obtain methyl 4-chloro-[1,1':2',1"-terphenyl]-2-carboxylate (138.6 g, yield 92.1%).

### <Step 1-2>

Methyl 4-chloro-[1,1':2',1"-terphenyl]-2-carboxylate (138.6 g, xxx.x mmol) was dissolved in THF (1000 mL), and the reaction mixture was cooled to 0°C using an ice bath. Thereafter, methylmagnesium bromide (3.0 M in diethyl ether, 357.8 mL, 1073.4 mmol) was added dropwise over 1 hour. The reaction mixture was then allowed to warm to room temperature and stirred for 3 hours. After completion of the reaction, the reaction mixture was cooled again to 0°C using an ice bath and quenched (neutralized) with an aqueous NH₄Cl solution. The resulting mixture was extracted with H₂O and ethyl acetate to separate the organic layer, which was then dried over MgSO₄ and filtered. The filtered organic layer was concentrated under reduced pressure, and the residue was purified by column chromatography to obtain 2-(4-chloro-[1,1':2',1"-terphenyl]-2-yl)propan-2-ol (131.4 g, 94.8% yield).

### <Step 1-3>

2-(4-chloro-[1,1':2',1"-terphenyl]-2-yl)propan-2-ol 131.4 g (407.0 mmol) was dissolved in Methylene chloride (600ml), and then mixture was cooled to 0°C using an ice bath. Thereafter, methanesulfonic acid 39.12 g(407.0 mmol) was slowly added dropwise over 15 minutes. After removal of the ice bath, the reaction mixture was stirred at 40°C using an oil bath. After completion of the reaction, the mixture was neutralized with a cooled aqueous K₂CO₃ solution and extracted with methylene chloride. The extracted organic layer was dried over MgSO4, filtered, and concentrated under reduced pressure. The residue was purified by column chromatography to obtain 2-chloro-9,9-dimethyl-5-phenyl-9H-fluorene (108.2 g, yield 87.2%).

### <Step 1-4>

2-chloro-9,9-dimethyl-5-phenyl-9H-fluorene 108.2 g(354.93 mmol), 4,4,4',4',5,5,5',5'-octamethyl-2,2'-bi(1,3,2-dioxaborolane) 108.2g(425.9 mmol), Pd(dppf) Cl₂ 7.8 g(10.7 mmol), XPhos 16.9 g(35.5 mmol), KOAc 104.5 g(1064.8 mmol) and 1000 ml of 1,4-Dioxane were added, and the mixture was stirred under reflux at 110°C for 8 hours. After completion of the reaction, the reaction mixture was cooled to room temperature and extracted with H2O and MC to afford an organic layer. The extracted organic layer was dried over MgSO₄, filtered, and concentrated under reduced pressure. The residue was purified by column chromatography to obtain Core 1 (116.5 g, yield 88.9%).

Mass : [(M+H)⁺] : 367

### [Preparation Example 2]

### Synthesis of Core 2

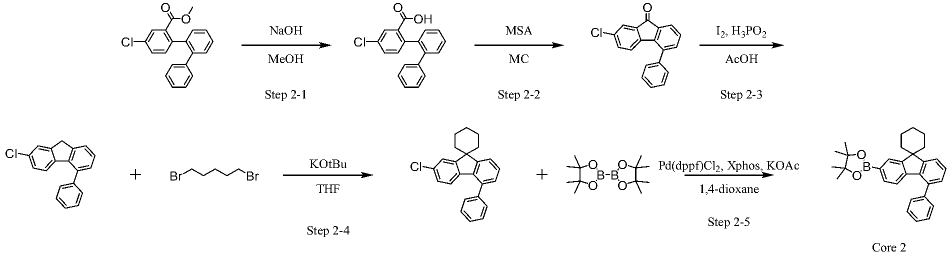

### <Step 2-1>

methyl 4-chloro-[1,1':2',1"-terphenyl]-2-carboxylate 80.0 g(247.84 mmol), NaOH 99.14 g(2478.4 mmol), 160 ml of MeOH, and 1600 ml of H₂O were added, and the mixture was stirred under reflux for 8 hours. After completion of the reaction, MeOH was removed under reduced pressure, and the residue was acidified by adding 800 ml of 6N HCl. The resulting solid was filtered and washed with H2O. the solid was dried in an oven to obtain 4-chloro-[1,1':2',1"-terphenyl]-2-carboxylic acid (74.5 g, yield : 97.3%).

### <Step 2-2>

4-chloro-[1,1' :2',1' '-terphenyl]-2-carboxylic acid 74.5 g was subjected to the same manner as in Step 1-3 of Preparation Example 1 to obtain 2-chloro-5-phenyl-9H-fluoren-9-one (66.5 g, yield : 94.8%).

### <Step 2-3>

I₂ 21.5g (84.56 mmol) was added 400 ml of acetic acid and stirred. 50% aqueous H₃PO₂ solution (54 g, 409.9 mmol) was then added, and the mixture was refluxed with stirring. Thereafter, 4-chloro-[1,1':2',1"-terphenyl]-2-carboxylic acid (74.5 g, 256.23 mmol), obtained in Step 2-2, was dissolved in acetic acid (570 mL) and added dropwise to the reaction mixture over 30 minutes to 1 hour. The resulting mixture was then refluxed with stirring for 24 hours. After completion of the reaction, the reaction mixture was cooled to room temperature and extracted with water and toluene. The extracted organic layer was concentrated under reduced pressure, and the residue was purified by column chromatography to obtain 2-chloro-5-phenyl-9H-fluorene (62.6 g, yield : 88.3%).

### <Step 2-4>

2-chloro-5-phenyl-9H-fluorene 62.6 g(226.2 mmol) was dissolved in 600 ml of THF with stirring, and the mixture was cooled to 0°C using an ice bath. KOtBu (63.5 g, 565.5 mmol) was added dropwise over 15 minutes, and the mixture was stirred for 30 minutes. Subsequently, 1,5-dibromopentane (130.0 g, 565.5 mmol) was added dropwise over 30 minutes. The reaction mixture was then allowed to warm to room temperature and stirred for 8 hours. After completion of the reaction, the organic layer was extracted using H₂O and methylene chloride, and then dried over MgSO₄ and filtered. The filtered organic layer was concentrated under reduced pressure, and the residue was purified by column chromatography to obtain 2'-chloro-5'-phenylspiro[cyclohexane-1,9'-fluorene] (59.2 g, yield : 75.9%).

### <Step 2-5>

Core 2 (55.6 g, 74.2% yield) was obtained in the same manner as in Step 1-4 of Preparation Example 1, except that 2'-chloro-5'-phenylspiro[cyclohexane-1,9'-fluorene] (59.2 g, 171.6 mmol) was used as the starting material.

Mass : [(M+H)⁺] : 367

### [Preparation Example 3]

### Synthesis of Core 3

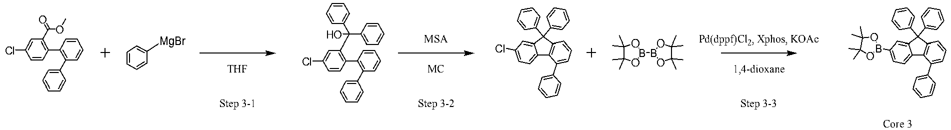

Core 3 (43.9 g, 45.4% yield over three steps) was obtained in the same manner as in Step 1-2, Step 1-3, and Step 1-4 of Preparation Example 1 for Step 3-1, Step 3-2, and Step 3-3, respectively, except that methyl 4-chloro-[1,1':2',1"-terphenyl]-2-carboxylate 60.0 g(185.9 mmol) and phenylmagnesium bromide(1M in THF) 464.7 ml(464.7 mmol) were used as the starting materials.

Mass : [(M+H)⁺] : 521

### [Preparation Example 4]

### Synthesis of Core 4

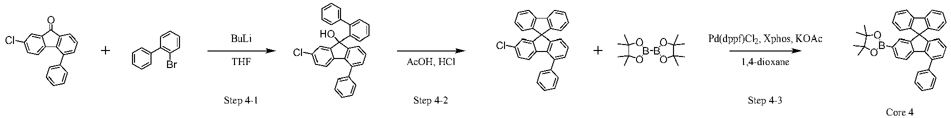

### <Step 4-1>

2-chloro-5-phenyl-9H-fluoren-9-one 50 g(171.9 mmol) was dissolved in 500 ml of THF and cooled to -78°C using a dry ice/acetone bath. In a separate reactor, 2-bromo-1,1'-biphenyl 40.1 g(171.9 mmol) was dissolved in 400 ml of THF and cooled to - 78°C using a dry ice/acetone bath. n-BuLi (1.6 M in hexane, 75.7 mL, 189.17 mmol) was then slowly added dropwise over 1 hour. The mixture was stirred at -78°C for 2 hours. The resulting solution containing 2-bromo-1,1'-biphenyl was then transferred dropwise via cannula to the reaction mixture containing 2-chloro-5-phenyl-9H-fluoren-9-one at -78°C. After completion of the addition, the reaction mixture was allowed to warm to room temperature and stirred for 3 hours.

After completion of the reaction, the mixture was extracted with H₂O and ethyl acetate to afford an organic layer. The extracted organic layer was dried over MgSO₄, filtered, and the filtrate was concentrated under reduced pressure. The residue was purified by column chromatography to obtain 9-([1,1'-biphenyl]-2-yl)-2-chloro-5-phenyl-9H-fluoren-9-ol (66.3 g, yield : 86.7%).

### <Step 4-2>

9-([1,1'-biphenyl]-2-yl)-2-chloro-5-phenyl-9H-fluoren-9-ol 66.3 g(149.1 mmol) was added to 650 ml of AcOH and 150 ml of 6N HCl, and the mixture was stirred under reflux for 4 hours. After completion of the reaction, the resulting solid was filtered and washed with water. The solid was dissolved in methylene chloride, dried over MgSO₄, and filtered. The filtrate was recrystallized from methylene chloride and MeOH, and the resulting solid was filtered and washed with MeOH. The solid was dried in an oven to obtain 2-chloro-5-phenyl-9,9'-spirobi[fluorene] (58.1 g, yield : 91.3%).

### <Step 4-3>

Core 4 (57.0 g, yield : 80.8%) was obtained in the same manner as in Step 1-4 of Preparation Example 1, except that 2-chloro-5-phenyl-9,9'-spirobi[fluorene] 58.1 g(188.6 mmol) was used as the starting material.

Mass : [(M+H)⁺] : 519

### <Synthesis Examples 1 to 22>

### [Synthesis Example 1] Synthesis of Compound 4

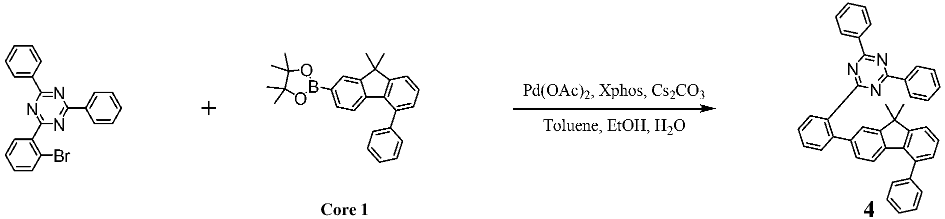

2-(2-bromophenyl)-4,6-diphenyl-1,3,5-triazine 8.0 g(leq, 20.6 mmol), Core 1 (8.0 g, 1.05eq, 21.6 mmol) of Preparation Example 1, Pd(OAc)₂ 0.1 g(0.03eq, 0.6 mmol), Cs₂CO₃ 20.1 g (3.0eq, 61.8 mmol), and Xphos 0.6 g(0.06eq, 1.2 mmol) were added to 100 ml of Toluene, 25 ml of EtOH and 25 ml of H₂O, and the resulting mixture was heated to reflux for 4 hours. After the reaction was completed, the reaction mixture was extracted with Methylene chloride, and the extracted organic layer was dried over MgSO₄ and filtered, and the filtrate was concentrated under reduced pressure. The residue was purified by column chromatography using dichloromethane and hexane as eluents, and recrystallized from toluene and acetone to obtain Compound 4 (7.8 g, yield 65.3 %).

Mass : [(M+H)⁺] : 578

### [Synthesis Example 2] Synthesis of Compound 5

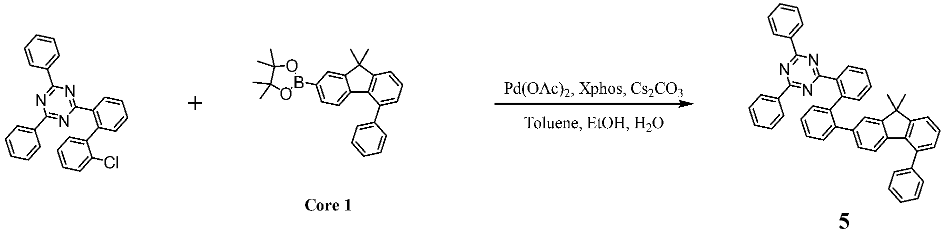

Compound 5 (11.0 g, yield 70.9%) was obtained in the same manner as Synthesis Example 1, except that 2-(2'-chloro-[1,1'-biphenyl]-2-yl)-4,6-diphenyl-1,3,5-triazine 10.0 g(leq, 23.8 mmol) was used as the starting material, while using the same equivalent ratios for the remaining reagents.

Mass : [(M+H)⁺] : 655

### [Synthesis Example 3] Synthesis of Compound 6

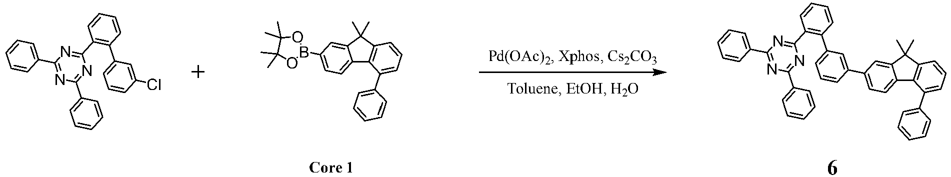

Compound 6 (12.3 g, yield 78.9%) was obtained in the same manner as Synthesis Example 1, except that 2-(3'-chloro-[1,1'-biphenyl]-2-yl)-4,6-diphenyl-1,3,5-triazine (10.0 g, 23.8 mmol) was used as the starting material, while using the same equivalent ratios for the remaining reagents.

Mass : [(M+H)⁺] : 655

### [Synthesis Example 4] Synthesis of Compound 9

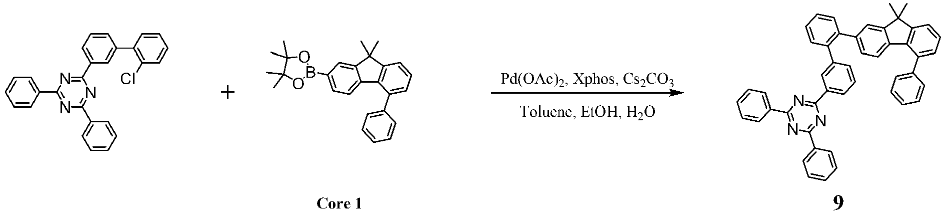

Compound 9 (11.1 g, yield 71.6%) was obtained in the same manner as Synthesis Example 1, except that 2-(2'-chloro-[1,1'-biphenyl]-3-yl)-4,6-diphenyl-1,3,5-triazine (10.0 g, 23.8 mmol) was used as the starting material, while using the same equivalent ratios for the remaining reagents.

Mass : [(M+H)⁺] : 655

### [Synthesis Example 5] Synthesis of Compound 10

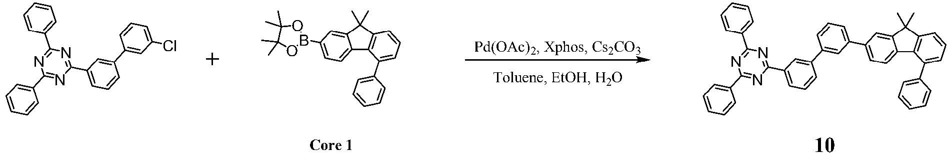

Compound 10 (13.2 g, yield 85.1%) was obtained in the same manner as Synthesis Example 1, except that 2-(3'-chloro-[1,1'-biphenyl]-3-yl)-4,6-diphenyl-1,3,5-triazine (10.0 g, 23.8 mmol) was used as the starting material, while using the same equivalent ratios for the remaining reagents.

Mass : [(M+H)⁺] : 655

### [Synthesis Example 6] Synthesis of Compound 13

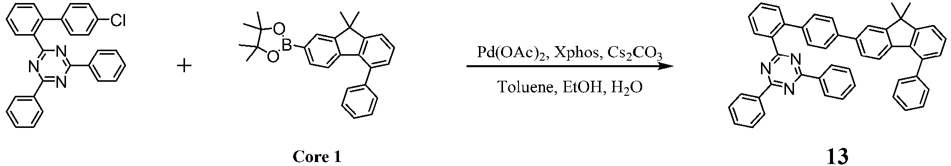

Compound 13 (10.6 g, yield 68.3%) was obtained in the same manner as Synthesis Example 1, except that 2-(4'-chloro-[1,1'-biphenyl]-2-yl)-4,6-diphenyl-1,3,5-triazine (10.0 g, 23.8 mmol) was used as the starting material, while using the same equivalent ratios for the remaining reagents.

Mass : [(M+H)⁺] : 655

### [Synthesis Example 7] Synthesis of Compound 25

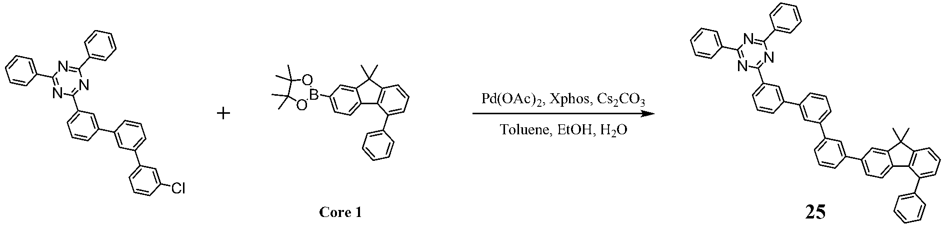

Compound 25 (10.8 g, yield 73.5%) was obtained in the same manner as Synthesis Example 1, except that 2-(3''-chloro-[1,1':3',1"-terphenyl]-3-yl)-4,6-diphenyl-1,3,5-triazine (10.0 g, 20.2 mmol) was used as the starting material, while using the same equivalent ratios for the remaining reagents.

Mass : [(M+H)⁺] : 730

### [Synthesis Example 8] Synthesis of Compound 29

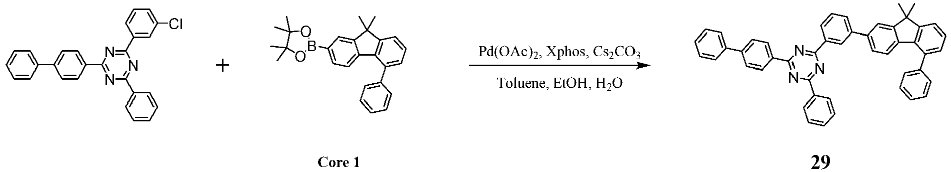

Compound 29 (14.1 g, yield 90.3%) was obtained in the same manner as Synthesis Example 1, except that 2-(4'-chloro-[1,1'-biphenyl]-2-yl)-4,6-diphenyl-1,3,5-triazine (10.0 g, 23.8 mmol) was used as the starting material, while using the same equivalent ratios for the remaining reagents.

Mass : [(M+H)⁺] : 655

### [Synthesis Example 9] Synthesis of Compound 49

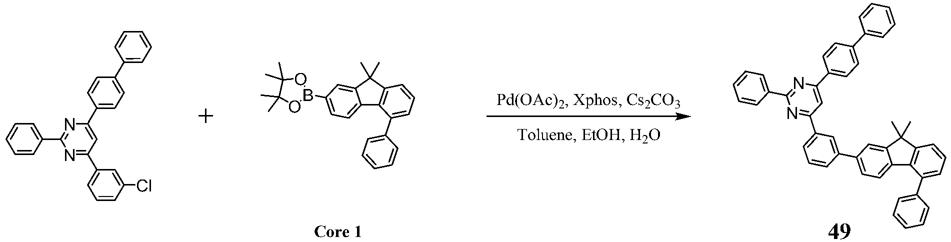

Compound 49 (14.6 g, yield 93.8%) was obtained in the same manner as Synthesis Example 1, except that 4-([1,1'-biphenyl]-4-yl)-6-(3-chlorophenyl)-2-phenylpyrimidine (10.0 g, 23.8 mmol) was used as the starting material, while using the same equivalent ratios for the remaining reagents.

Mass : [(M+H)⁺] : 654

### [Synthesis Example 10] Synthesis of Compound 58

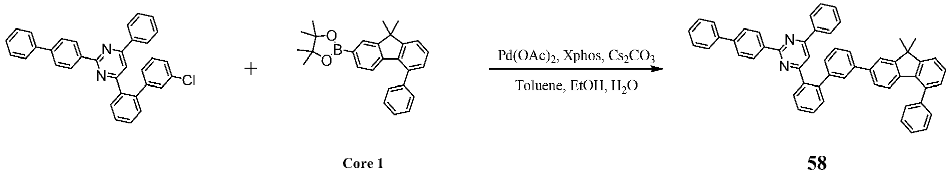

Compound 58 (12.4 g, yield 84.2%) was obtained in the same manner as Synthesis Example 1, except that 2-([1,1'-biphenyl]-4-yl)-4-(3'-chloro-[1,1'-biphenyl]-2-yl)-6-phenylpyrimidine (10.0 g, 20.2 mmol) was used as the starting material, while using the same equivalent ratios for the remaining reagents.

Mass : [(M+H)⁺] : 730

### [Synthesis Example 11] Synthesis of Compound 59

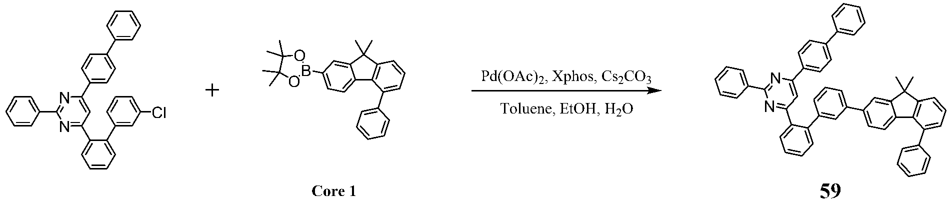

Compound 59 (11.3 g, yield 76.9%) was obtained in the same manner as Synthesis Example 1, except that 4-([1,1'-biphenyl]-4-yl)-6-(3'-chloro-[1,1'-biphenyl]-2-yl)-2-phenylpyrimidine (10.0 g, 20.2 mmol) was used as the starting material, while using the same equivalent ratios for the remaining reagents.

Mass : [(M+H)⁺] : 730

### [Synthesis Example 12] Synthesis of Compound 60

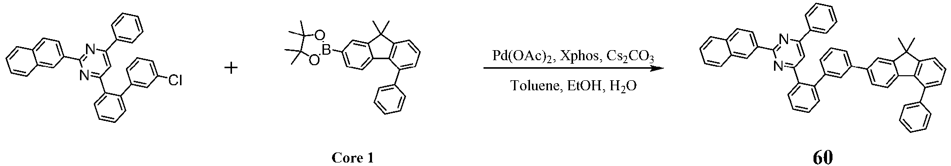

Compound 60 (10.2 g, yield 68.2%) was obtained in the same manner as Synthesis Example 1, except that 4-(3'-chloro-[1,1'-biphenyl]-2-yl)-2-(naphthalen-2-yl)-6-phenylpyrimidine (10.0 g, 21.3 mmol) was used as the starting material, while using the same equivalent ratios for the remaining reagents.

Mass : [(M+H)⁺] : 704

### [Synthesis Example 13] Synthesis of Compound 90

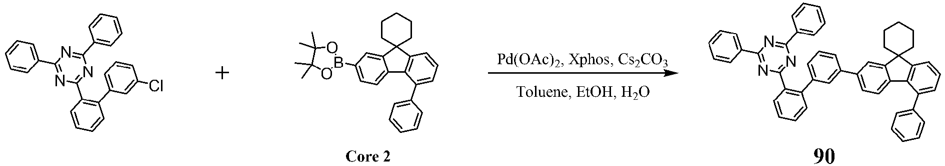

2-(3'-chloro-[1,1'-biphenyl]-2-yl)-4,6-diphenyl-1,3,5-triazine 10.0 g(leq, 23.8 mmol), Core 2 10.9 g(1.05eq, 25.0 mmol) of Preparation Example 2, Pd(OAc)₂ 0.2 g(0.03eq, 0.7 mmol), Cs₂CO₃ 23.3 g(3.0eq, 71.4 mmol), and Xphos 0.7 g(0.06eq, 1.4 mmol) were added to 100 ml of Toluene, 25 ml of EtOH, and 25 ml of H₂O, and the resulting mixtures was heated to reflux for 4 hours. After the reaction was completed, the reaction mixture was extracted with Methylene chloride, and the extracted organic layer was dried over MgSO₄ and filtered, and the filtrate was concentrated under reduced pressure. The residue was purified by column chromatography using dichloromethane and hexane as eluents, and recrystallized from toluene and acetone to obtain Compound 90 (11.9 g, yield 72.3 %).

Mass : [(M+H)⁺] : 695

### [Synthesis Example 14] Synthesis of Compound 105

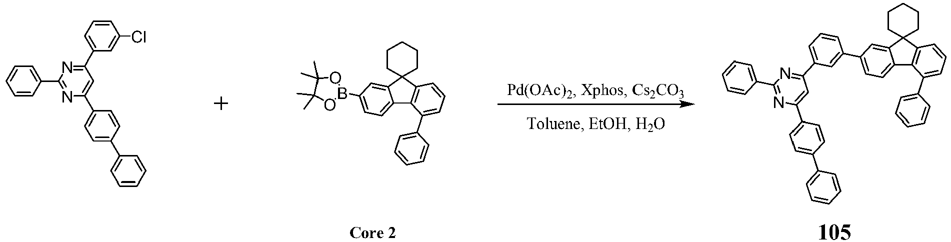

Compound 105 (13.4 g, yield 80.8%) was obtained in the same manner as Synthesis Example 13, except that 4-([1,1'-biphenyl]-4-yl)-6-(3-chlorophenyl)-2-phenylpyrimidine (10.0 g, 23.9 mmol) was used as the starting material, while using the same equivalent ratios for the remaining reagents.

Mass : [(M+H)⁺] : 694

### [Synthesis Example 15] Synthesis of Compound 126

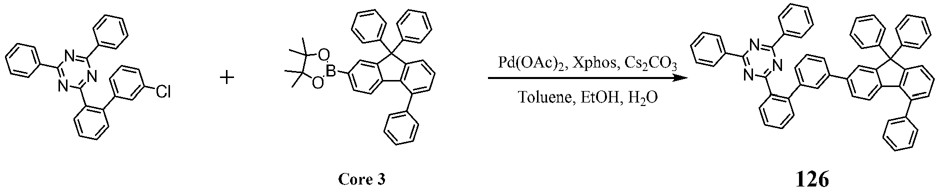

2-(3'-chloro-[1,1'-biphenyl]-2-yl)-4,6-diphenyl-1,3,5-triazine 10.0 g(leq, 23.8 mmol), Core 3 13.0 g(1.05eq, 25.0 mmol) of Preparation Example 3, Pd(OAc)₂ 0.2 g(0.03eq, 0.7 mmol), Cs₂CO₃ 23.3 g(3.0eq, 71.4 mmol), and Xphos 0.7 g(0.06eq, 1.4 mmol) were added to 100 ml of Toluene, 25 ml of EtOH, and 25 ml of H₂O, and the resulting mixture was heated to reflux for 4 hours. After the reaction was completed, the reaction mixture was extracted with Methylene chloride, and the extracted organic layer was dried over MgSO₄ and filtered, and the filtrate was concentrated under reduced pressure. The residue was purified by column chromatography using dichloromethane and hexane as eluents, and recrystallized from toluene and acetone to obtain Compound 126 (18.5 g, yield 74.9 %).

Mass : [(M+H)⁺] : 779

### [Synthesis Example 16] Synthesis of Compound 162

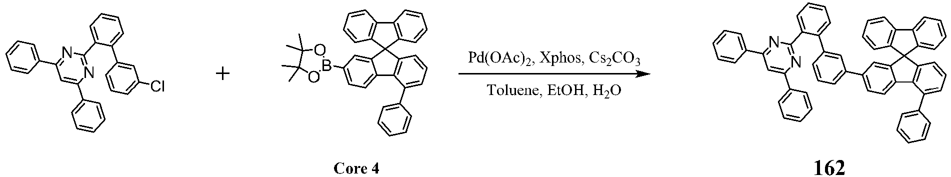

2-(3'-chloro-[1,1'-biphenyl]-2-yl)-4,6-diphenylpyrimidine 10.0 g(1eq, 23.9 mmol), Core 4 13.0 g(1.05eq, 25.1 mmol) of Preparation Example 4, Pd(OAc)₂ 0.2 g(0.03eq, 0.7 mmol), Cs₂CO₃ 23.3 g(3.0eq, 71.6 mmol), and Xphos 0.7 g(0.06eq, 1.4 mmol) were added to 100 ml of Toluene, 25 ml of EtOH, and 25 ml of H₂O, and the resulting mixture was heated to reflux for 4 hours. After the reaction was completed, the reaction mixture was extracted with Methylene chloride, and the extracted organic layer was dried over MgSO₄ and filtered, and the filtrate was concentrated under reduced pressure. The residue was purified by column chromatography using dichloromethane and hexane as eluents, and recrystallized from toluene and acetone to obtain Compound 162 (11.6 g, yield 62.9 %).

Mass : [(M+H)⁺] : 776

### [Synthesis Example 17] Synthesis of Compound 174

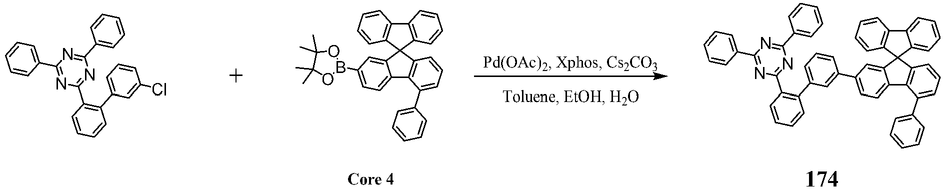

Compound 174 (16.3 g, yield 88.2%) was obtained in the same manner as Synthesis Example 16, except that 2-(3'-chloro-[1,1'-biphenyl]-2-yl)-4,6-diphenyl-1,3,5-triazine (10.0 g, 23.8 mmol) was used as the starting material, while using the same equivalent ratios for the remaining reagents.

Mass : [(M+H)⁺] : 777

### [Synthesis Example 18] Synthesis of Compound 193

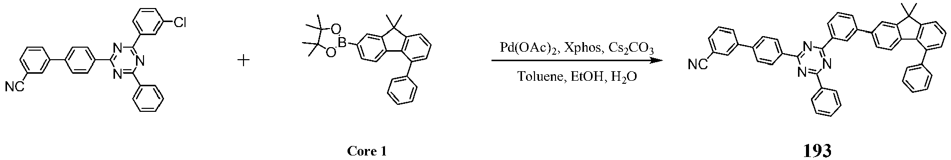

Compound 193 (8.5 g, yield 55.6%) was obtained in the same manner as Synthesis Example 1, except that 4'-(4-(3-chlorophenyl)-6-phenyl-1,3,5-triazin-2-yl)-[1,1'-biphenyl]-3-carbonitrile (10.0 g, 22.5 mmol) was used as the starting material, while using the same equivalent ratios for the remaining reagents.

Mass : [(M+H)⁺] : 680

### [Synthesis Example 19] Synthesis of Compound 196

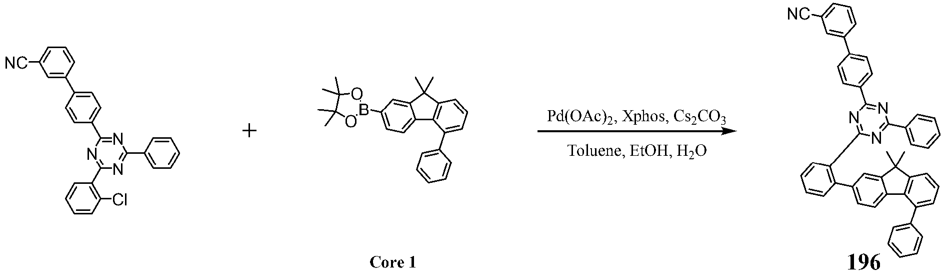

Compound 196 (7.8 g, yield 51.2%) was obtained in the same manner as Synthesis Example 1, except that 4'-(4-(2-chlorophenyl)-6-phenyl-1,3,5-triazin-2-yl)-[1,1'-biphenyl]-3-carbonitrile (10.0 g, 22.5 mmol) was used as the starting material, while using the same equivalent ratios for the remaining reagents.

Mass : [(M+H)⁺] : 680

### [Synthesis Example 20] Synthesis of Compound 197

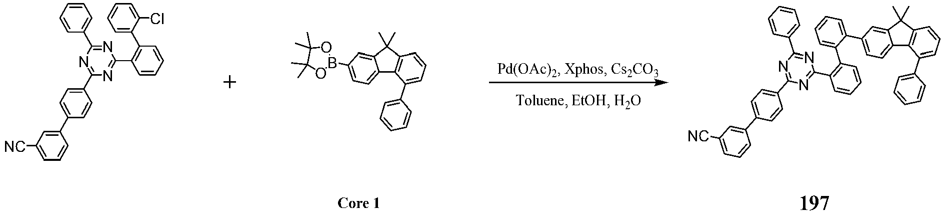

Compound 197 (8.5 g, yield 58.6%) was obtained in the same manner as Synthesis Example 1, except that 4'-(4-(2'-chloro-[1,1'-biphenyl]-2-yl)-6-phenyl-1,3,5-triazin-2-yl)-[1,1'-biphenyl]-3-carbonitrile (10.0 g, 19.2 mmol) was used as the starting material, while using the same equivalent ratios for the remaining reagents.

Mass : [(M+H)⁺] : 756

### [Synthesis Example 21] Synthesis of Compound 203

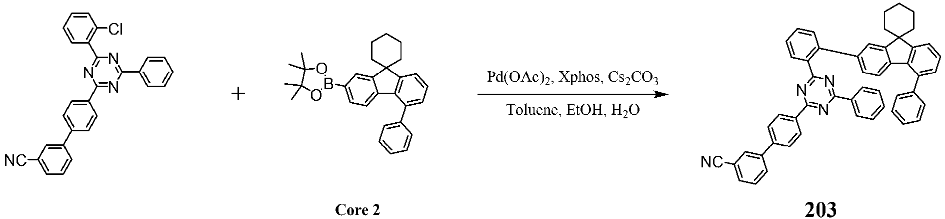

Compound 203 (10.4 g, yield 62.2%) was obtained in the same manner as Synthesis Example 1, except that 4'-(4-(2-chlorophenyl)-6-phenyl-1,3,5-triazin-2-yl)-[1,1'-biphenyl]-3-carbonitrile (10.0 g, 22.5 mmol) was used as the starting material, while using the same equivalent ratios for the remaining reagents.

Mass : [(M+H)⁺] : 720

### [Synthesis Example 22] Synthesis of Compound 204

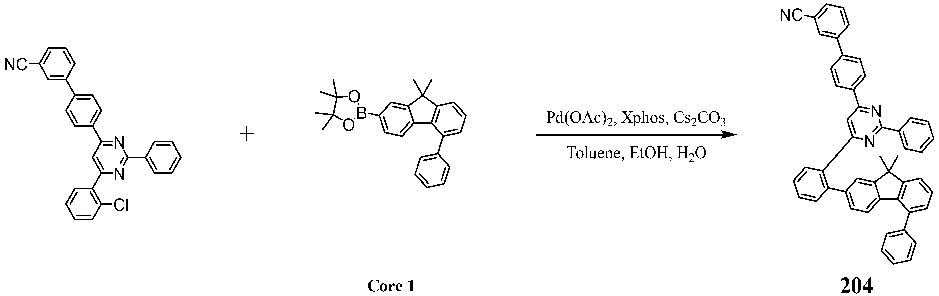

Compound 204 (8.8 g, yield 57.6%) was obtained in the same manner as Synthesis Example 1, except that 4'-(6-(2-chlorophenyl)-2-phenylpyrimidin-4-yl)-[1,1'-biphenyl]-3-carbonitrile (10.0 g, 22.5 mmol) was used as the starting material, while using the same equivalent ratios for the remaining reagents.

Mass : [(M+H)⁺] : 679

### [Reference Example]

The structures of compounds HT-1, HAT-CN, HT-2, BH, BD, LiQ, ET-1, ET-2, ET-3, ET-4, ET-5, and ET-6 used in Examples 1 to 22 and Comparative Examples 1 to 6 below are as follows, respectively.

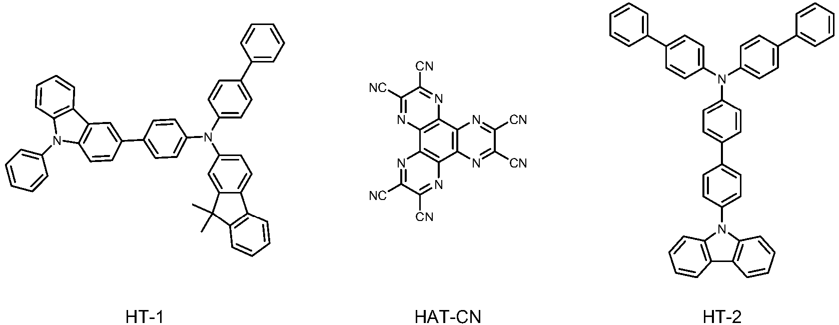

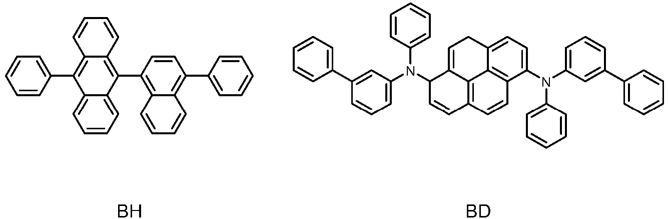

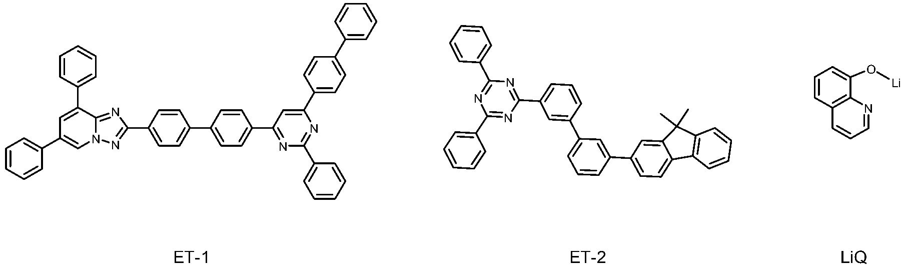

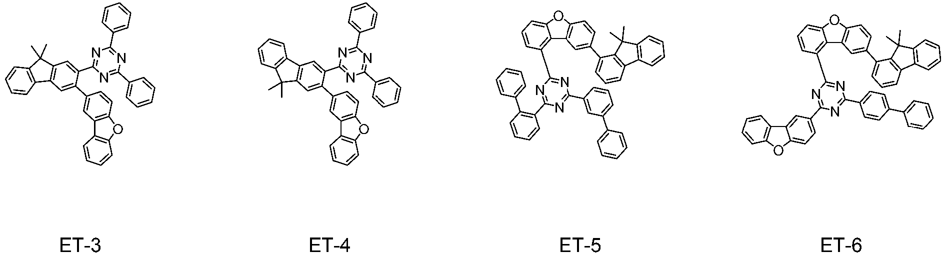

### [Examples 1 to 5] Manufacturing of Blue organic EL device

The compounds synthesized in the above synthesis examples were subjected to high-purity sublimation purification in a conventionally known method, and then blue organic EL device (OLED) was manufactured by using the compound as an electron transport layer material as follows.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å) / HT-1 (1400 Å) / HT-2 (50 Å) / BH + 2% BD (200 Å) / ET-2 (50 Å) / each of Compounds 193, 196, 192, 203, and 204 : LiQ = 1:1 (300 Å) / LiF (10 Å ) / Al (1000 Å) were stacked in order to manufacture an organic EL device.

### [Comparative Example 1] Manufacturing of blue organic EL device

A blue organic EL device of Comparative Example 1 was manufactured in the same manner as Example 1, except that ET-1 was deposited to a thickness of 300 Å as an electron transport layer material instead of Compound 193.

### [Evaluation example 1]

For each of the blue organic EL devices manufactured in Examples (Ex.) 1 to 5 and Comparative Example 1, a driving voltage, a current efficiency at a current density of 10 mA/cm², and an emission peak were measured, and the results are shown in Table 1 below.

**[Table 1]**

| Sample | Electron transport layer | Driving voltage | EL peak | Current efficiency |
|---|---|---|---|---|
| | | (V) | (nm) | (cd/A) |
| Ex. 1 | Compound 193 | 4.4 | 459 | 6.5 |
| Ex. 2 | Compound 196 | 4.2 | 461 | 6.7 |
| Ex. 3 | Compound 197 | 4.3 | 461 | 6.6 |
| Ex. 4 | Compound 203 | 4.2 | 460 | 6.7 |
| Ex. 5 | Compound 204 | 4.5 | 459 | 6.8 |
| Comp. Ex. 1 | ET-1 | 4.7 | 460 | 6.0 |

As shown in Table 1, it was appreciated that the blue organic EL devices of Examples 1 to 5 using the compound according to the present invention as an electron transport layer material exhibit significantly excellent performance in terms of driving voltage, emission peak and current efficiency compared to the blue organic EL device of Comparative Example 1 using conventional compound ET-1 as an electron transport layer material.

### [Examples 6 to 22] Manufacturing of Blue organic EL device

The compounds synthesized in the above synthesis examples were subjected to high-purity sublimation purification in a conventionally known method, and then blue organic EL device (OLED) was manufactured by using the compound as an electron transport auxiliary layer material as follows.

A glass substrate thin-film-coated with indium tin oxide (ITO) to a thickness of 1,500 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å) / HT-1 (1400 Å) / HT-2 (50 Å) / BH + 2% BD (200 Å) / each of Compounds 4 to 174 in Table 2/ ET-1 : LiQ = 1:1 (300 Å) / LiF (10 Å ) / Al (1000 Å) were stacked in order to manufacture an organic EL device.

### [Comparative Examples 2 to 6] Manufacturing of blue organic EL device

A blue organic EL devices of Comparative Examples 2 to 6 were respectively manufactured in the same manner as Example 6, except that Compounds ET-2 to ET-6, which are electron transport auxiliary layer materials, were deposited to a thickness of 50 Å instead of using Compound 4.

### [Evaluation example 2]

For each of the blue organic EL devices manufactured in Examples (Ex.) 6 to 22 and Comparative Examples (Comp. Ex.) 2 to 6, a driving voltage, a current efficiency at a current density of 10 mA/cm², and an emission peak were measured, and the results are shown in Table 2 below.

**[Table 2]**

| Sample | Electron transport auxiliary layer | Driving voltage | EL peak | Current efficiency |
|---|---|---|---|---|
| | | (V) | (nm) | (cd/A) |
| Ex. 6 | Compound 4 | 4.6 | 461 | 6.4 |
| Ex. 7 | Compound 5 | 4.6 | 460 | 6.6 |
| Ex. 8 | Compound 6 | 4.3 | 462 | 7.2 |
| Ex. 9 | Compound 9 | 4.2 | 460 | 7.1 |
| Ex. 10 | Compound 10 | 4.0 | 458 | 6.9 |
| Ex. 11 | Compound 13 | 4.4 | 459 | 6.6 |
| Ex. 12 | Compound 25 | 4.2 | 460 | 6.7 |
| Ex. 13 | Compound 29 | 4.5 | 460 | 6.4 |
| Ex. 14 | Compound 49 | 4.1 | 462 | 6.9 |
| Ex. 15 | Compound 58 | 4.0 | 462 | 6.4 |
| Ex. 16 | Compound 59 | 4.3 | 461 | 7.0 |
| Ex. 17 | Compound 60 | 4.4 | 459 | 6.6 |
| Ex. 18 | Compound 90 | 4.3 | 460 | 7.2 |
| Ex. 19 | Compound 105 | 4.1 | 459 | 6.5 |
| Ex. 20 | Compound 126 | 4.1 | 461 | 6.8 |
| Ex. 21 | Compound 162 | 4.7 | 460 | 6.8 |
| Ex. 22 | Compound 174 | 4.1 | 462 | 6.9 |
| Comp. Ex. 2 | ET-2 | 4.7 | 460 | 6.3 |
| Comp. Ex. 3 | ET-3 | 5.1 | 461 | 4.8 |
| Comp. Ex. 4 | ET-4 | 5.2 | 460 | 4.4 |
| Comp. Ex. 5 | ET-5 | 5.4 | 462 | 4.4 |
| Comp. Ex. 6 | ET-6 | 6.1 | 460 | 4.1 |

As shown in Table 2, it was confirmed that the blue organic EL devices of Examples 6 to 22 using the compound according to the present invention as an electron transport auxiliary layer material exhibit significantly excellent performance in terms of driving voltage, emission peak and current efficiency compared to the blue organic EL device of Comparative Examples 2 to 6, which use compounds ET-2 to ET-6 as the electron transport auxiliary layer material, wherein ET-2 to ET-6 do not include a phenyl group introduced at the 5-position of the fluorene-based moiety, which is an essential configuration of the present invention.

## Claims

1. A compound represented by Chemical Formula 1 below: 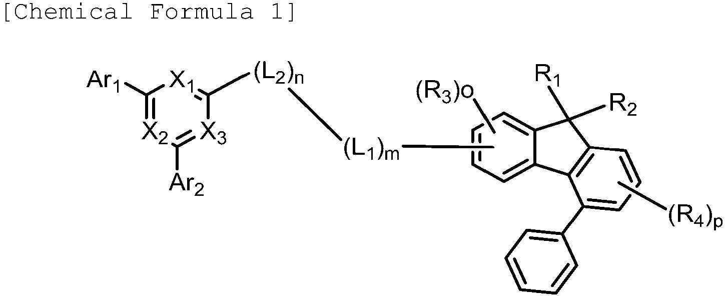 wherein in Chemical Formula 1,
X₁ to X₃ are the same as or different from each other and are each independently N or C(R₅), provided that at least two of X₁ to X₃ are N;
Ar₁ to Ar₂ are the same as or different from each other and are each independently selected from the group consisting of a hydrogen, a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group,
R₁ and R₂ are the same as or different from each other and are each independently selected from the group consisting of a hydrogen, a deuterium (D), a C₁-C₄₀ alkyl group, or a C₆-C₆₀ aryl group, or are bonded to each other to form a fused ring,
R₃ to R₅ are the same as or different from each other and are each independently selected from the group consisting of a hydrogen, a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group, or may be bonded to any adjacent group to form a fused ring,
L₁ and L₂ are the same as or different from one another and are each independently a single bond, or selected from the group consisting of a C₆ to C₁₈ arylene group and a heteroarylene group having 5 to 18 nuclear atoms,
n and m are each independently an integer of 0 to 2, provided that n + m ≥ 1, and
o and p are each independently an integer of 0 to 3, and
the arylene group, the heteroarylene group of L₁ and L₂; and the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, and arylamine group of Ar₁ to Ar₂, and R₁ to R₅ may be each independently substituted with at least one substituent selected from the group consisting of a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and wherein when the substituent is present in a plural number, they may be the same or different from each other.

2. The compound of Claim 1, wherein the ring containing X₁ to X₃ is any one selected from the following structural formulae: 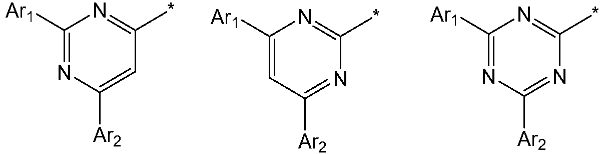 wherein the formulas,
* indicates a site where a bond with Chemical Formula 1 is made,
Ar₁ and Ar₂ are each as defined in claim 1.

3. The compound of Claim 1,
wherein Ar₁ and Ar₂ are the same as or different from each other, and each independently selected from the group consisting of a C₆ to C₆₀ aryl group, and a heteroaryl group having 5 to 60 nuclear atoms.

4. The compound of Claim 1,
wherein Ar₁ and Ar₂ are the same as or different from each other, and are each independently selected from the group consisting of the following structural formulae: 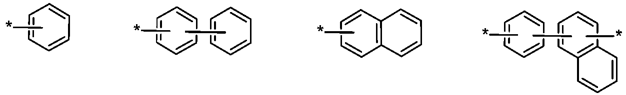 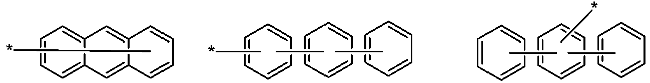 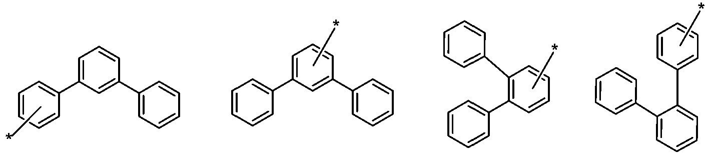 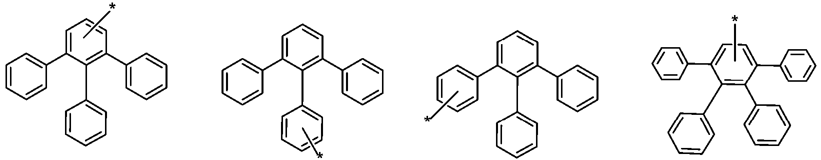 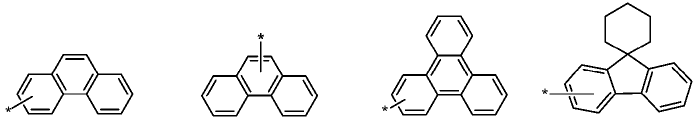 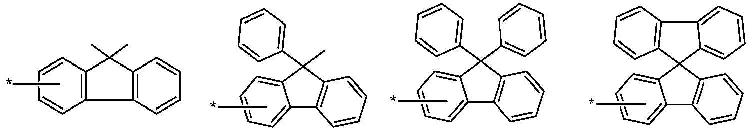 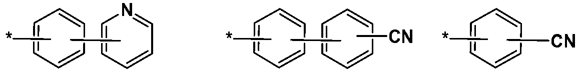 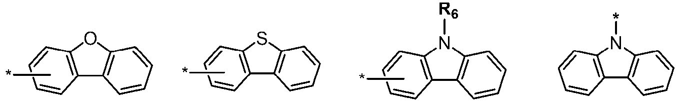 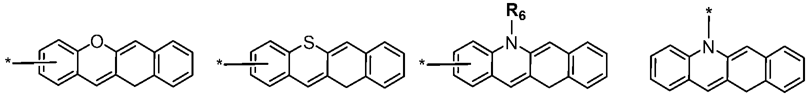 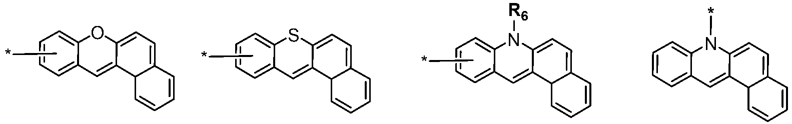 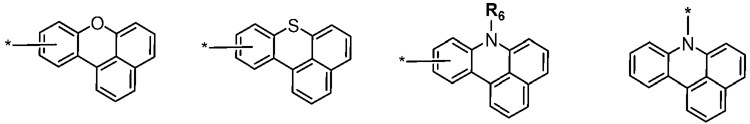 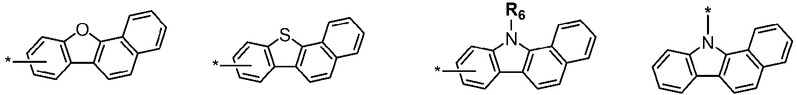 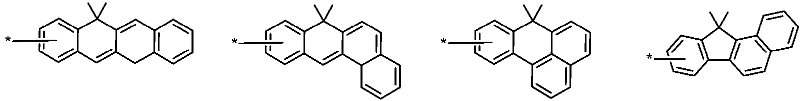
wherein the formulas,
* indicates a site where a bond with Chemical Formula 1 is made, and
R₆ is selected from the group consisting of a hydrogen, a deuterium (D), a C₁-C₄₀ alkyl group, a C₆-C₆₀ aryl group, and a heteroaryl group having 5 to 60 nuclear atoms.

5. The compound of Claim 1, wherein the moiety containing R₁ and R₂ is any one selected from group consisting of the following structural formulae: 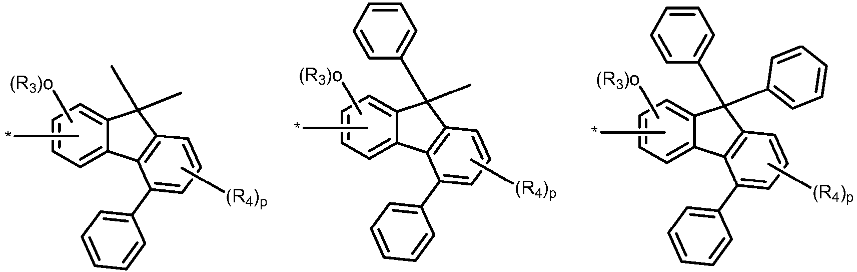 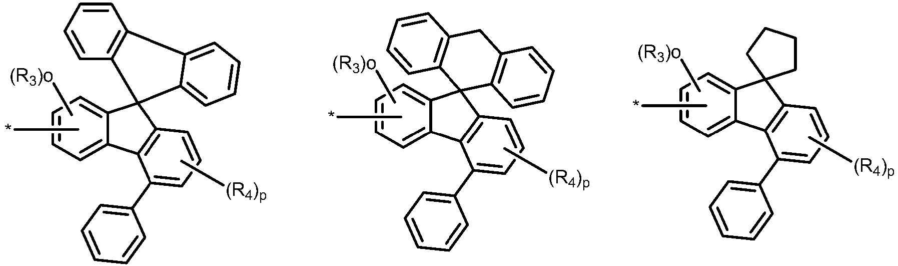 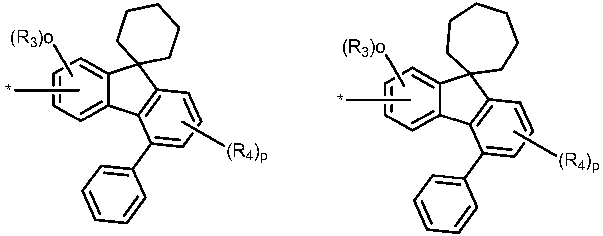 wherein the formulas,
* indicates a site where a bond with Chemical Formula 1 is made, and
R₃, R₄, O and p are each as defined in claim 1.

6. The compound of Claim 1, wherein L₁ and L₂ are the same as or different from each other, and are each independently selected from the group consisting of the following structural formulae: 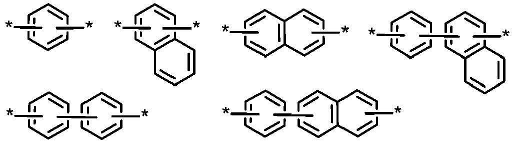 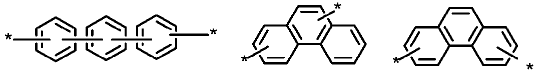 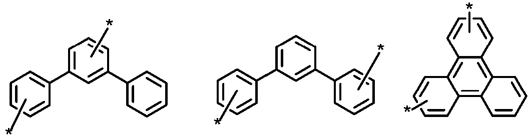 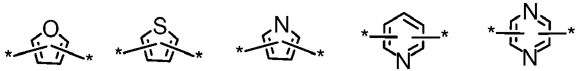 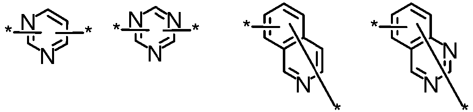 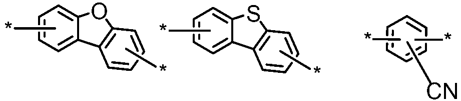 wherein the formulas,
* indicates a site where a bond with Chemical Formula 1 is made.

7. The compound of Claim 1, wherein the compound represented by Chemical Formula 1 is a compound represented by Chemical Formula 2 or Chemical Formula 3 below: 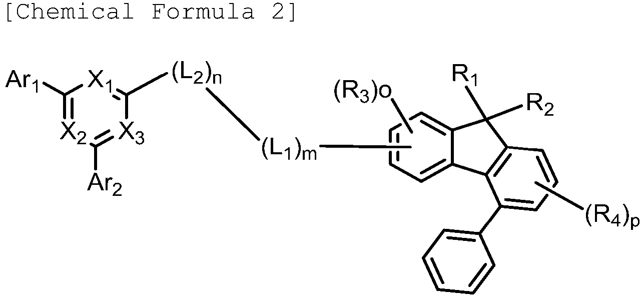 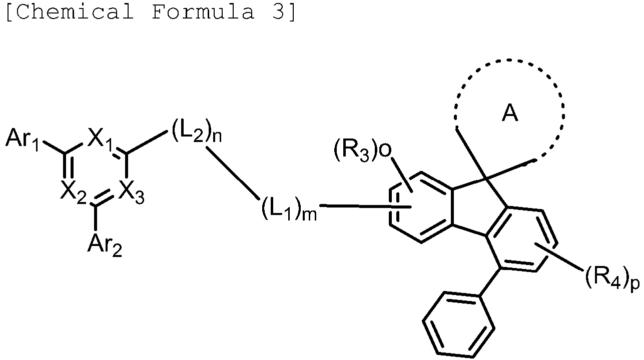 wherein the formulas,
Ring A is a C₅ to C₁₈ monocyclic or polycyclic hydrocarbon ring group, which may or may not include a heteroatom,
X₁ to X₃, Ar₁ to Ar₂, L₁ to L₂, R₁ to R₄, n, m, o and p are each as defined in claim 1, provided that cases where R₁ and R₂ are simultaneously hydrogen are excluded.

8. The compound of Claim 1, wherein the compound represented by Chemical Formula 1 is a compound represented by any one of Chemical Formulas 4 to Chemical Formula 7 below: 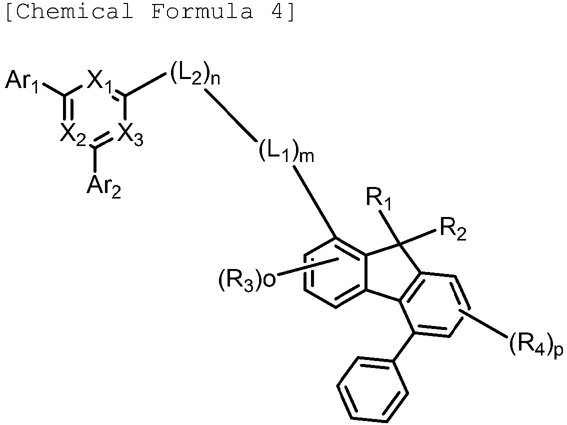 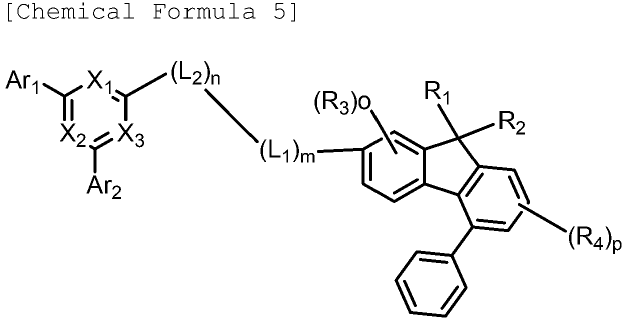 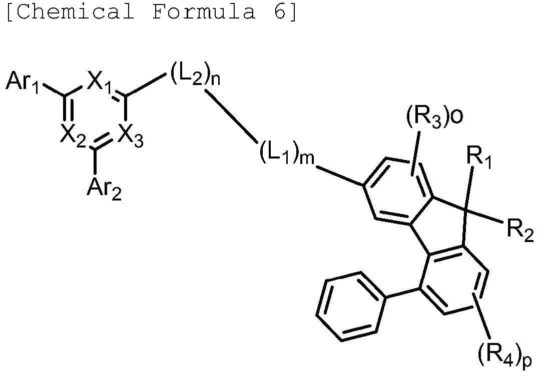 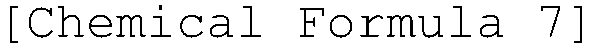 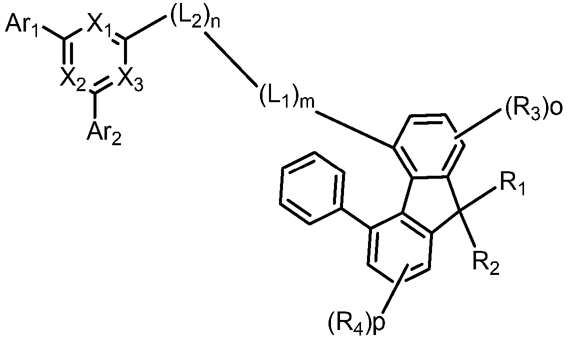 wherein the formulas,
X₁ to X₃, Ar₁ to Ar₂, L₁ to L₂, R₁ to R₄, n, m, o and p are each as defined in claim 1.

9. The compound of Claim 1, wherein the compound represented by Chemical Formula 1 is a compound represented by any one of Chemical Formulas 8 to Chemical Formula 15 below: 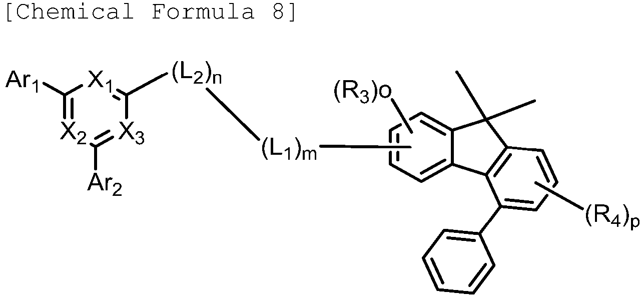 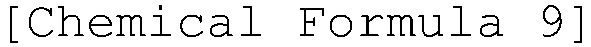 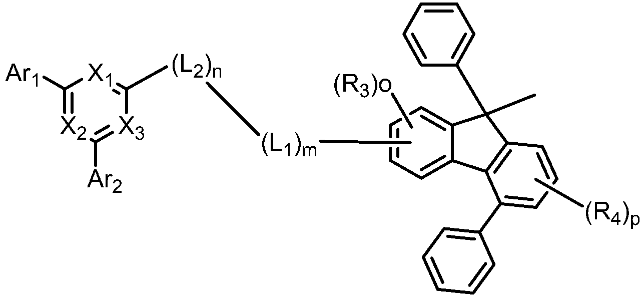 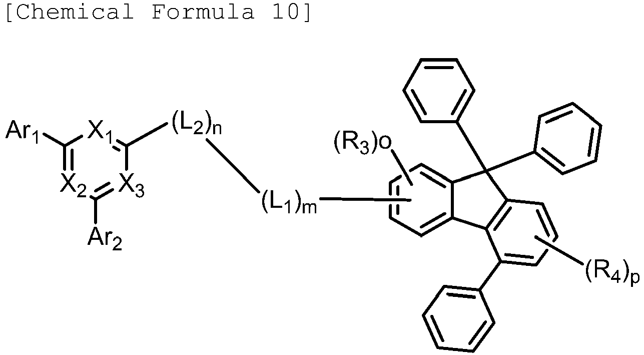 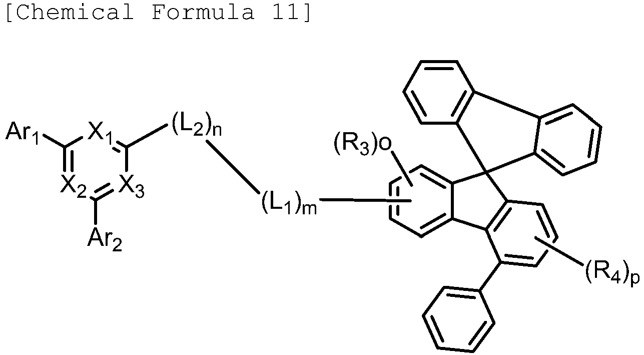 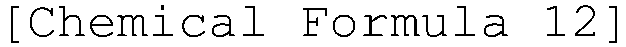 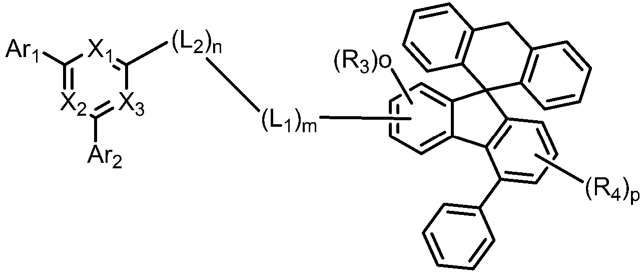 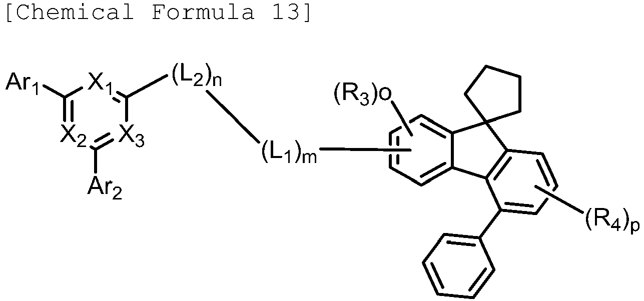 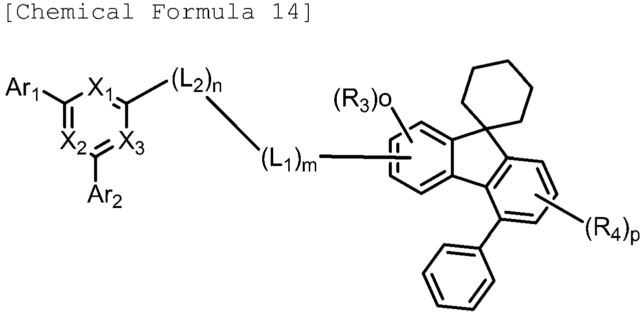 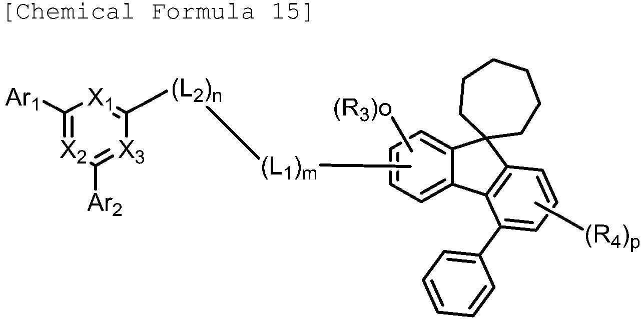 wherein the formulas,
X₁ to X₃, Ar₁ to Ar₂, L₁ to L₂, R₃ to R₄, n, m, o and p are each as defined in claim 1.

10. The compound of Claim 1, wherein the compound represented by Chemical Formula 1 is a compound represented by any one of Chemical Formulas 16 to Chemical Formula 18 below: 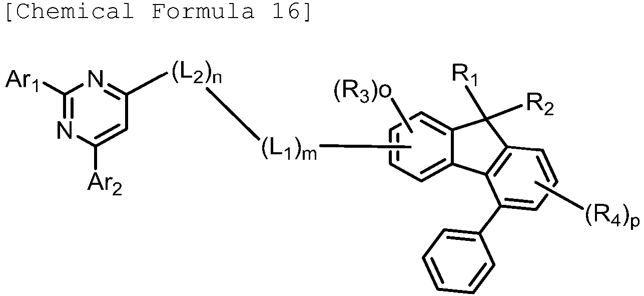 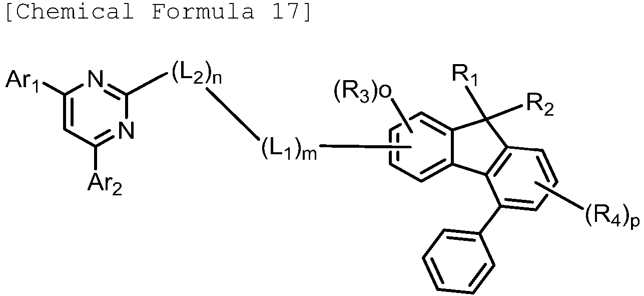 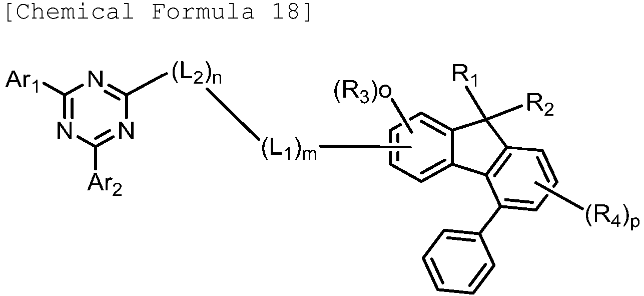 wherein the formulas,
Ar₁ to Ar₂, L₁ to L₂, R₁ to R₄, n, m, o and p are each as defined in claim 1.

11. The compound of Claim 1, wherein the compound represented by Chemical Formula 1 is a compound represented by any one of Chemical Formulas 19 to Chemical Formula 23 below: 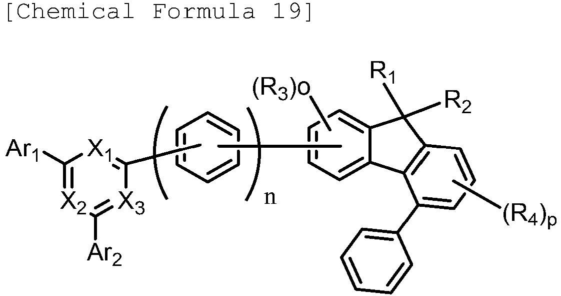 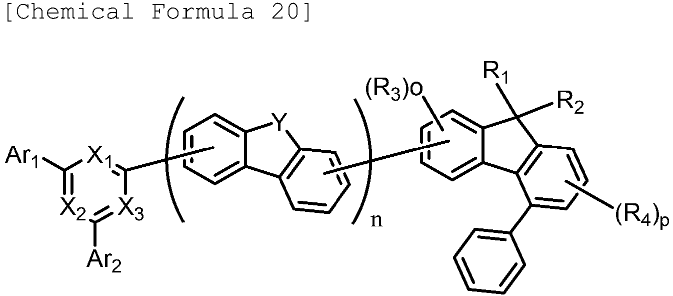 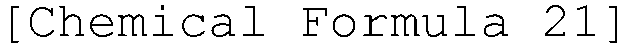 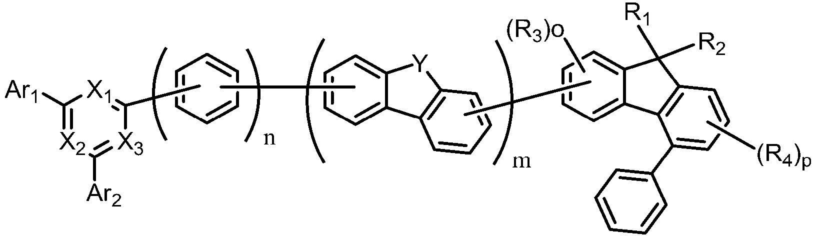 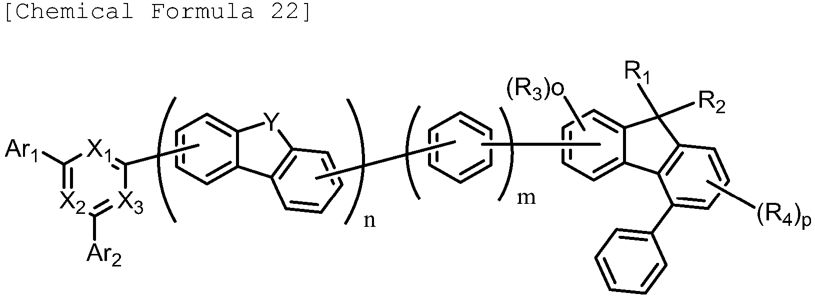 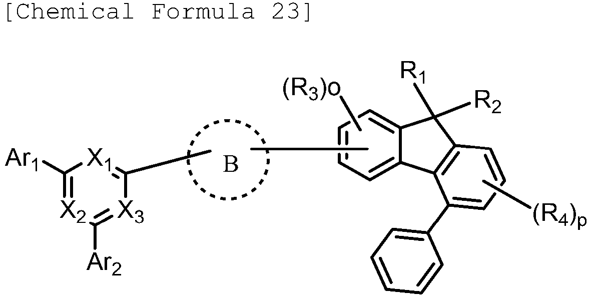 wherein the formulas,
Ring B is a divalent fused aromatic polycyclic group,
Y is O or S,
X₁ to X₃, Ar₁ to Ar₂, R₁ to R₄, n, m, o and p are each as defined in claim 1.

12. The compound of Claim 1, wherein the compound represented by Chemical Formula 1 is selected from compounds represented by any one of the following Chemical Formulas 1 to 208:

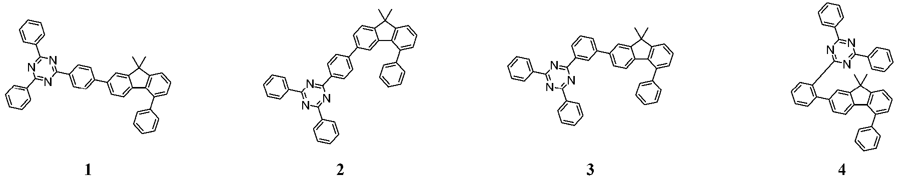

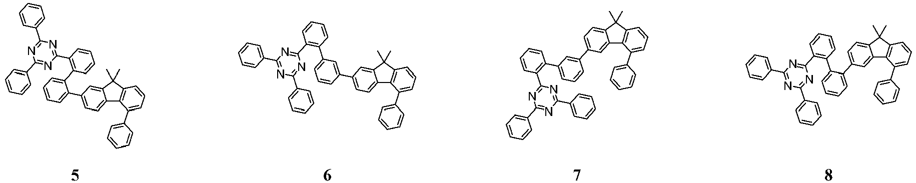

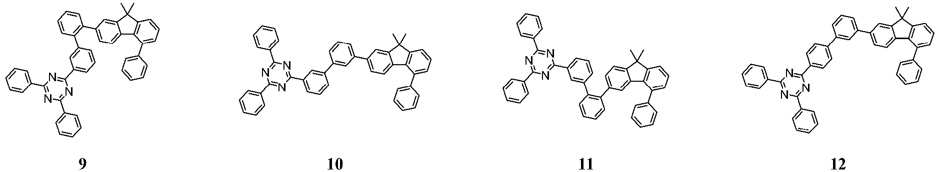

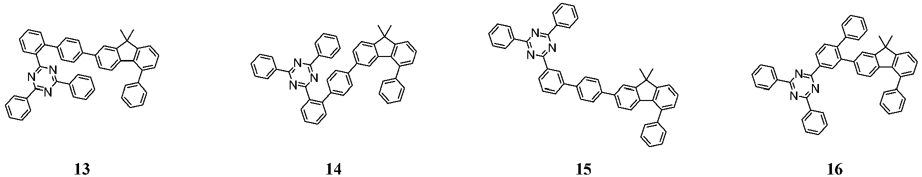

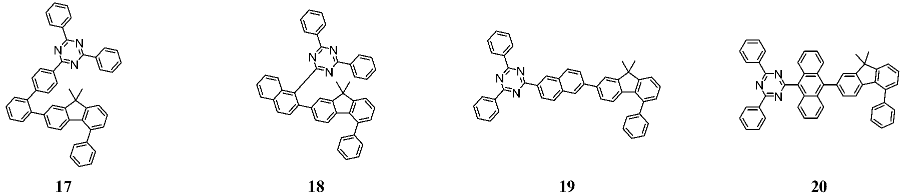

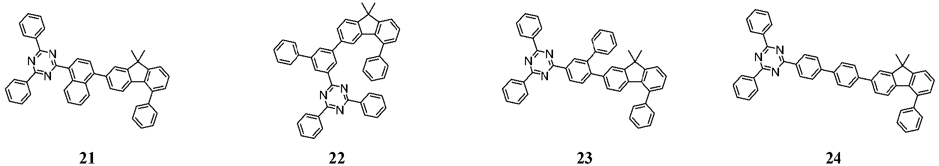

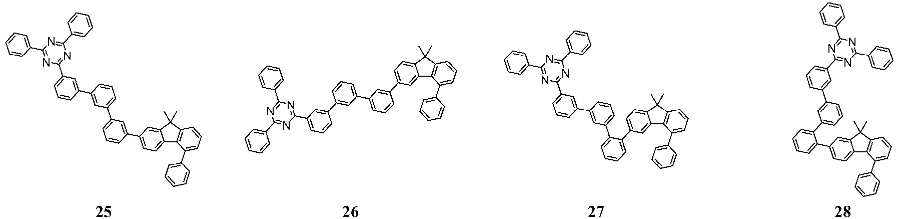

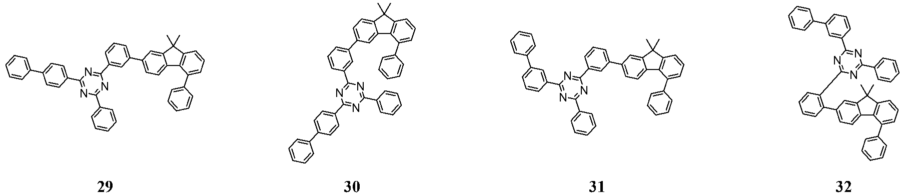

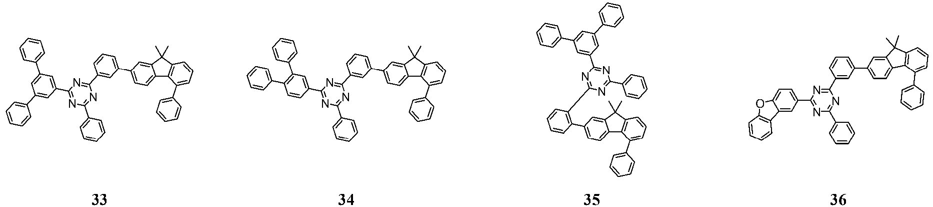

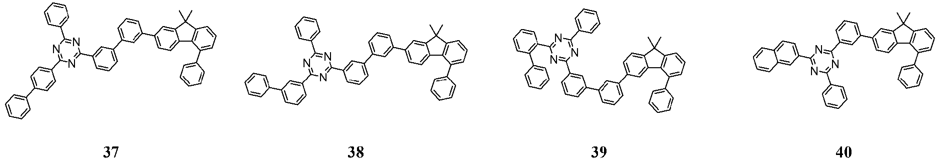

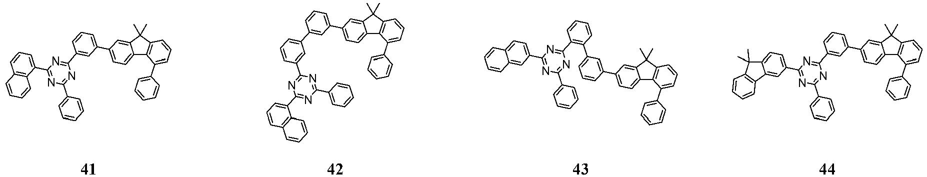

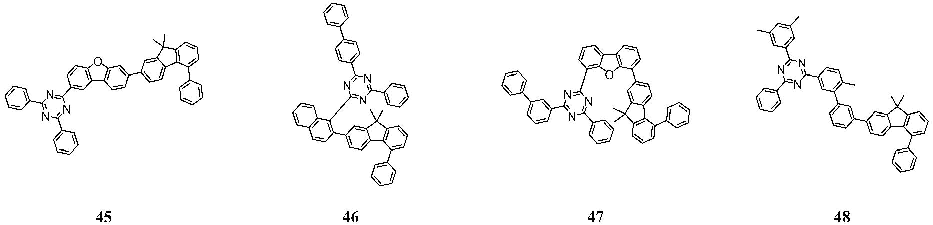

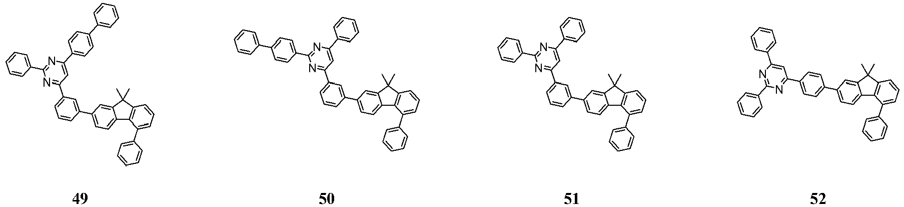

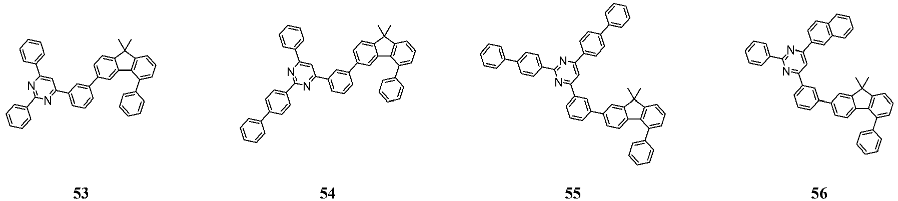

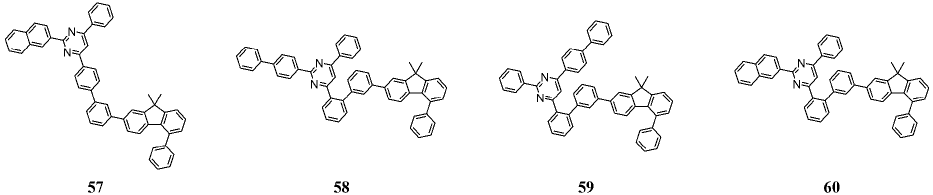

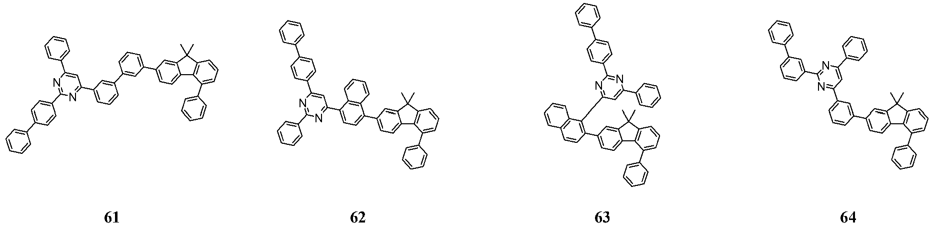

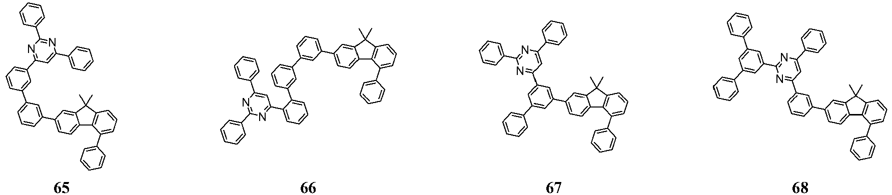

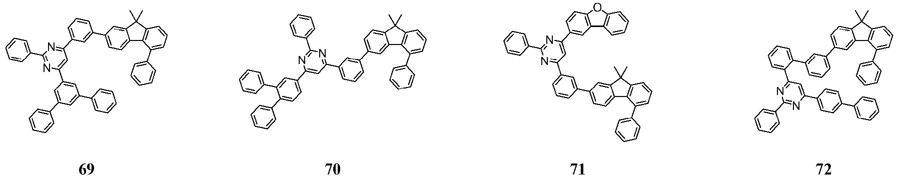

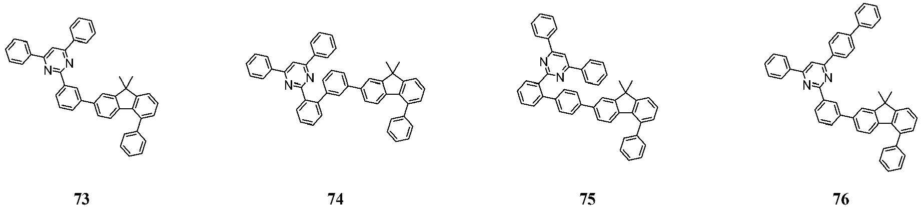

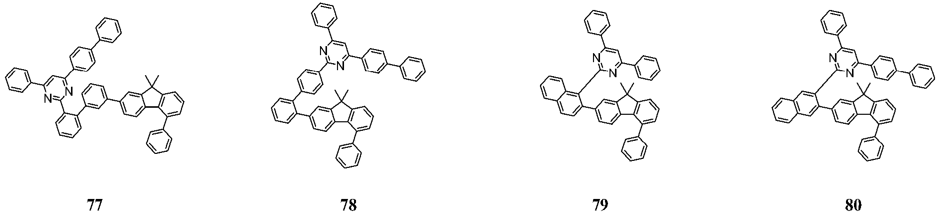

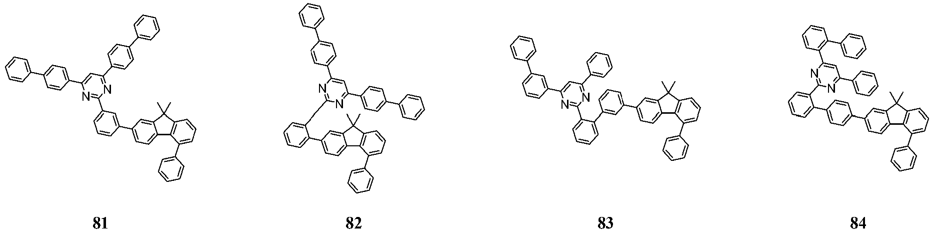

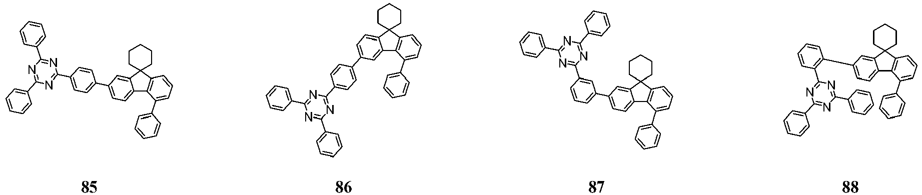

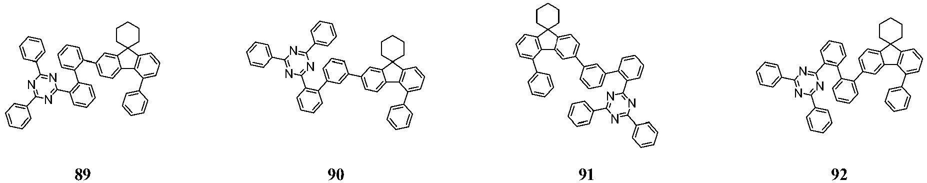

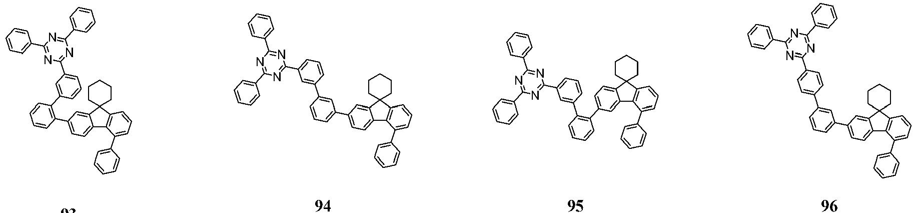

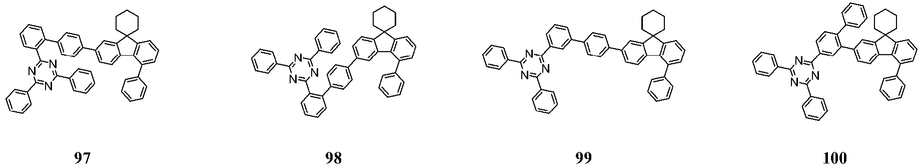

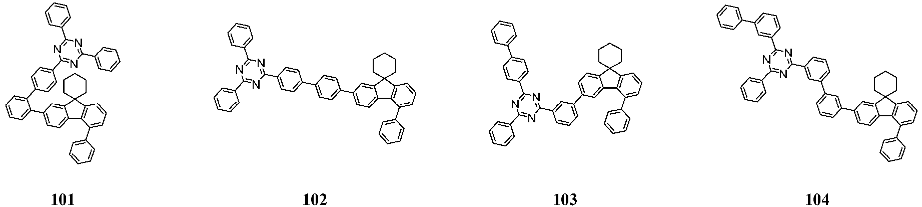

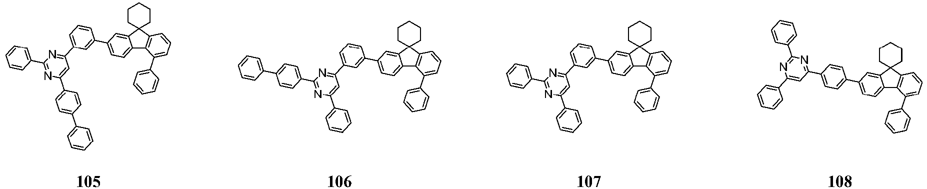

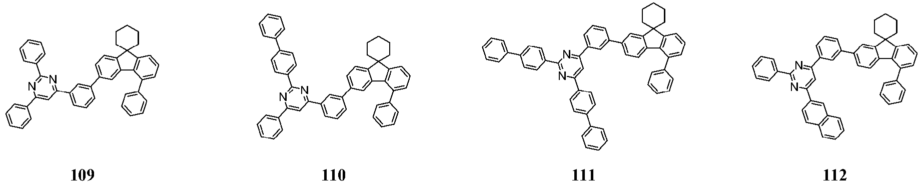

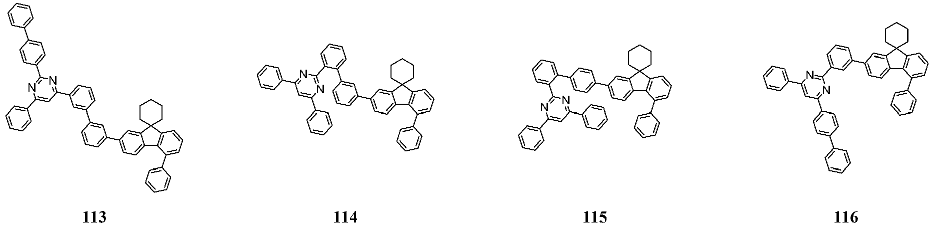

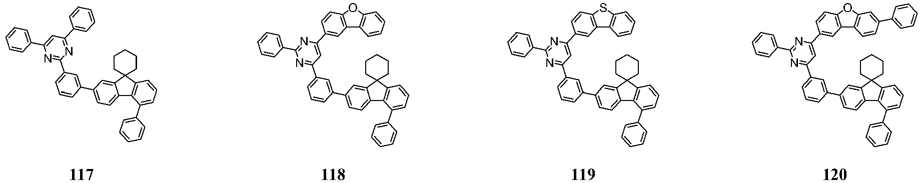

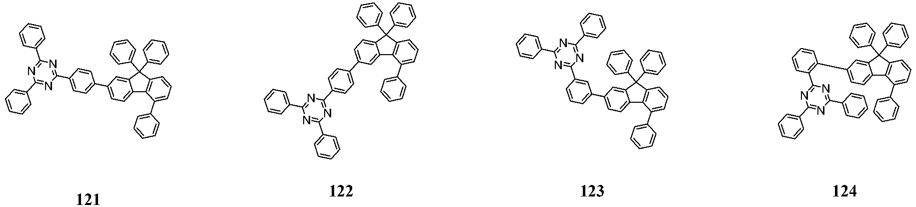

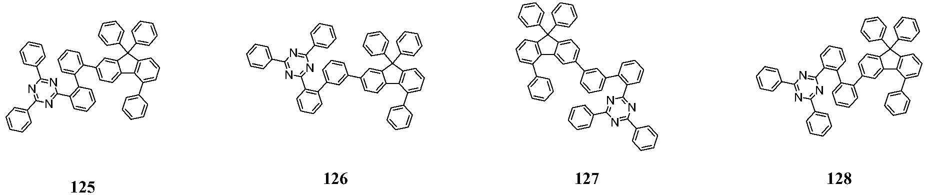

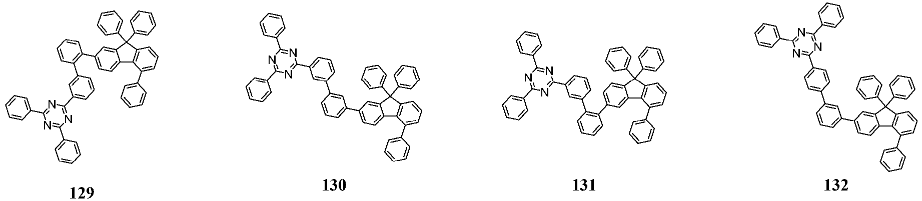

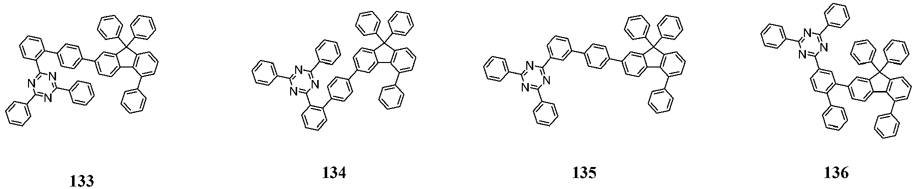

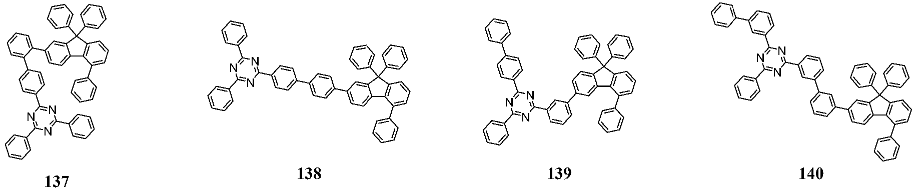

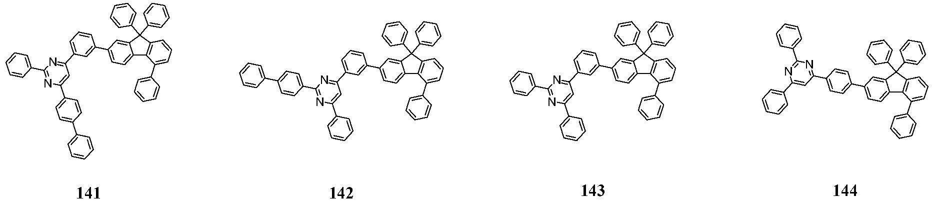

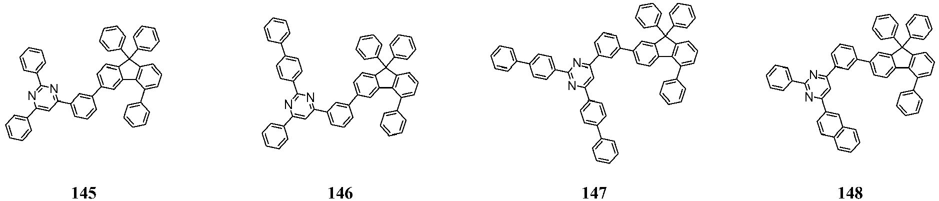

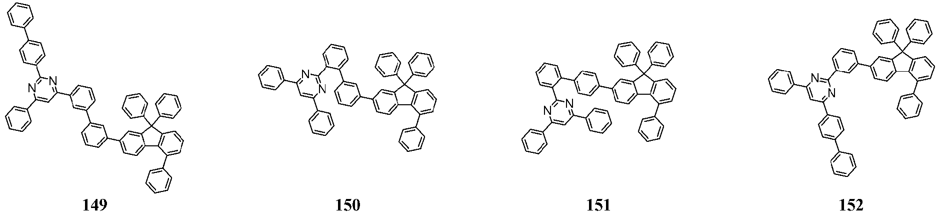

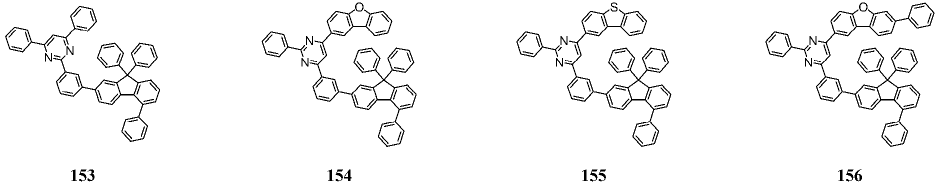

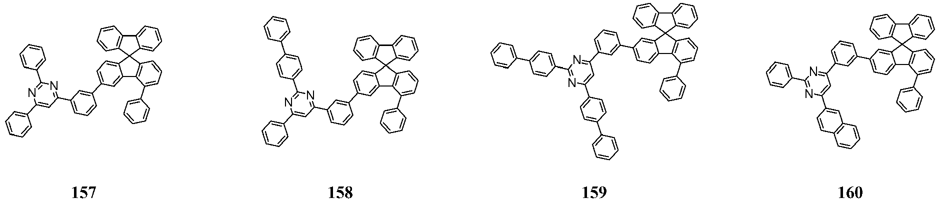

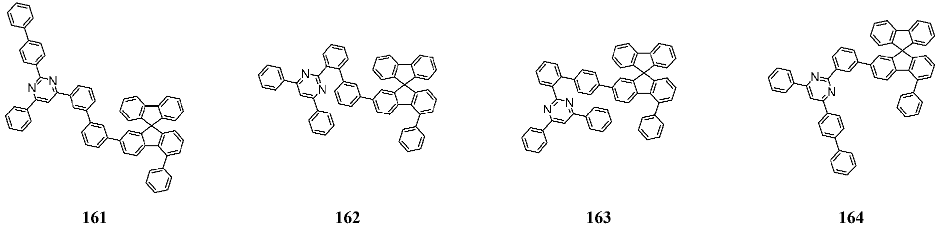

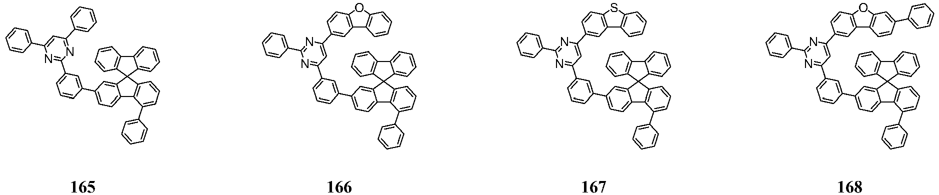

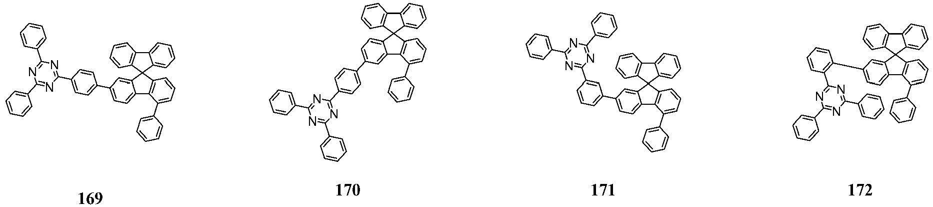

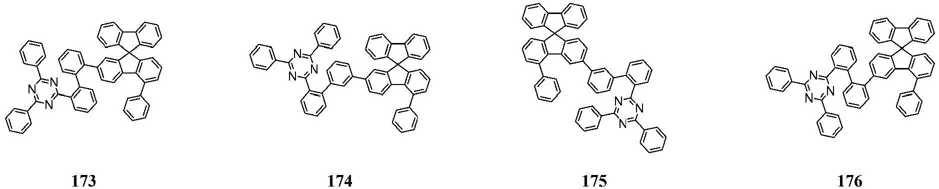

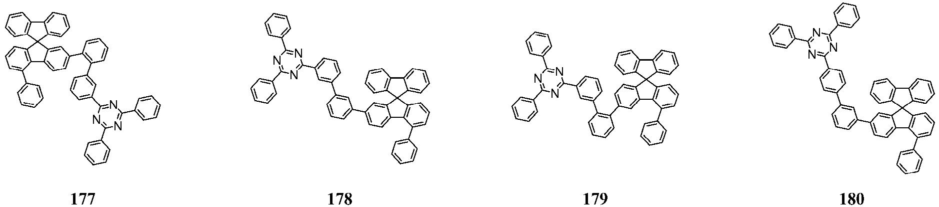

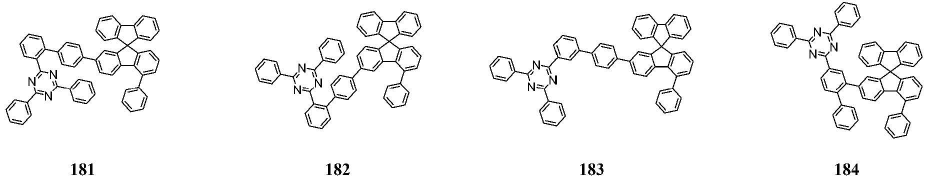

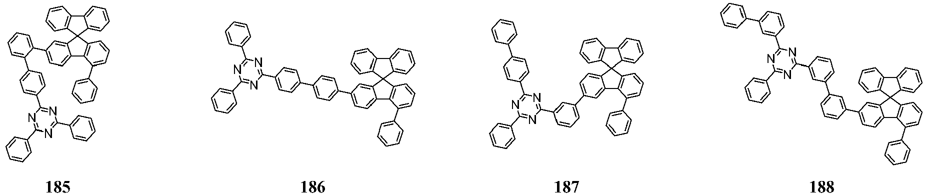

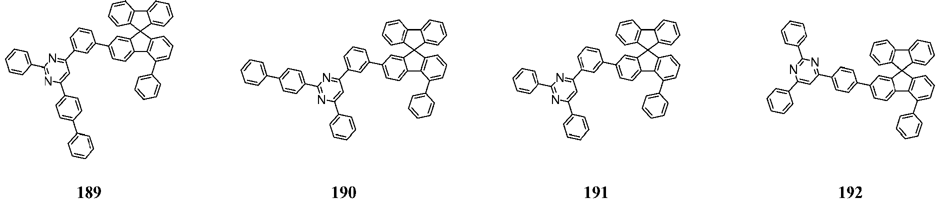

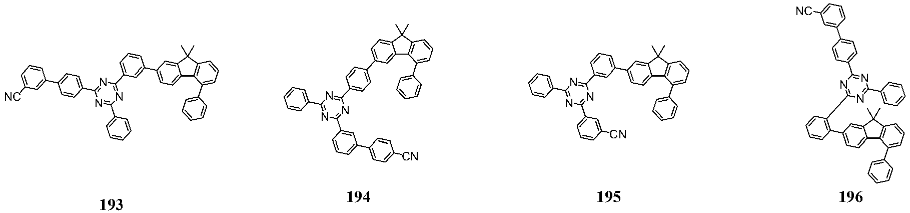

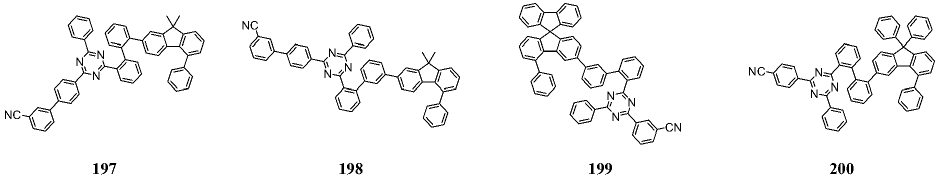

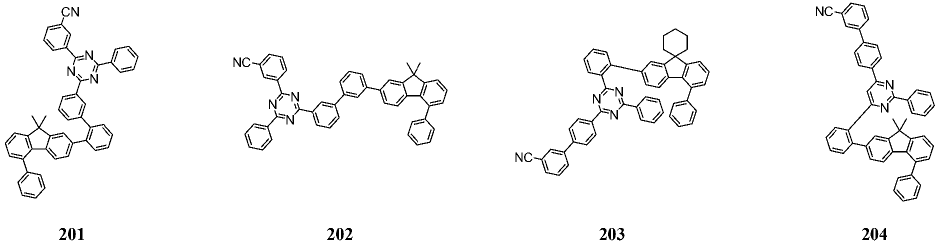

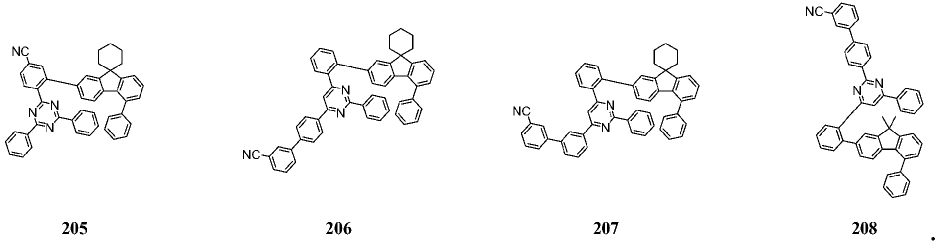

13. The compound of Claim 1, wherein the compound represented by Chemical Formula 1 is used as a material of a light emitting layer, a light emitting auxiliary layer, an electron transport layer, or an electron transport auxiliary layer.

14. An organic electroluminescent device comprising:
an anode;
a cathode; and
one or more organic layers disposed between the anode and the cathode,
wherein at least one of the one or more organic layers comprises the compound according to any one of claims 1 to 13.

15. The organic electroluminescent device of claim 14, wherein the organic layer comprising the compound is selected from the group consisting of a light emitting layer, a light emitting auxiliary layer, a lifespan improvement layer, an electron transport layer, and an electron transport auxiliary layer.
